# EUROPEAN PATENT SPECIFICATION

(11) **EP 3 962 584 B1**
(45) Date of publication and mention of the grant of the patent: **31.07.2024**
(21) Application number: 20727445.7
(22) Date of filing: 27.04.2020
(51) Int. Cl.: A61N 1/36, A61N 1/05, A61N 1/372

(54) **ADJUSTMENT OF STIMULATION IN RESPONSE TO ELECTRODE ARRAY MOVEMENT IN A SPINAL CORD STIMULATOR SYSTEM**
ANPASSUNG DER STIMULATION IN REAKTION AUF ELEKTRODENANORDNUNGSBEWEGUNGEN BEI EINEM RÜCKENMARKSTIMULATOR
RÉGLAGE DE LA STIMULATION EN RÉPONSE À UN MOUVEMENT DE RÉSEAU D'ÉLECTRODES DANS UN SYSTÈME DE STIMULATEUR DE MOELLE ÉPINIÈRE

(30) Priority: 30.04.2019 US 201962840534 P
(43) Date of publication of application: 09.03.2022
(73) Proprietor: Boston Scientific Neuromodulation Corporation, Valencia, CA 91355 (US)
(72) Inventor: ZHANG, Tianhe, Studio City, CA 91604 (US); ESTELLER, Rosana, Santa Clarita, CA 91390 (US); MOFFITT, Michael, A., Saugus, CA 91390 (US); BOCEK, Joseph, M., Seattle, WA 98102 (US)
(74) Representative: Vossius & Partner Patentanwälte Rechtsanwälte mbB
(86) International application number: PCT/US2020/030107
(87) International publication number: WO 2020/223165

(56) References cited:
- WO-A2-2006/107848
- WO-A2-2012/125863
- US-A1- 2014 163 639
- US-A1- 2015 360 031
- US-A1- 2018 078 769

## Description

### FIELD OF THE INVENTION

This application relates to Implantable Medical Devices (IMDs), and more specifically to techniques for providing stimulation in implantable neurostimulation systems.

### INTRODUCTION

Implantable neurostimulator devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a spinal cord stimulation (SCS) system, such as that disclosed in U.S. Patent 6,516,227. However, the present invention may find applicability with any implantable neurostimulator device system.

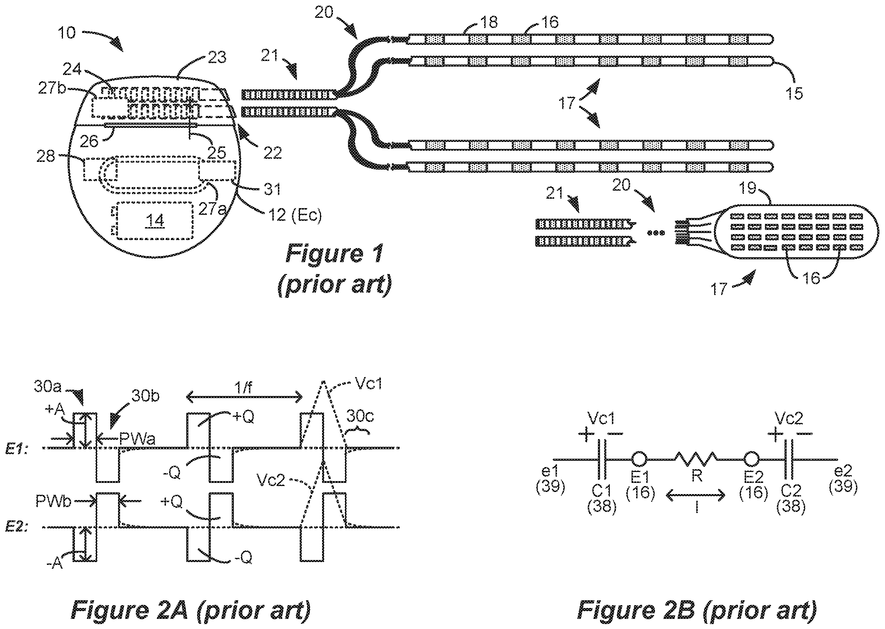

An SCS system typically includes an Implantable Pulse Generator (IPG) 10 shown in Figure 1. The IPG 10 includes a biocompatible conductive device case 12 that holds the IPG's circuitry and a battery 14 for providing power for the IPG to function. The IPG 10 is coupled to tissue-stimulating electrodes 16 via one or more electrode leads that form an electrode array 17. For example, one or more percutaneous leads 15 can be used having ring-shaped or split-ring electrodes 16 carried on a flexible body 18. In another example, a paddle lead 19 provides electrodes 16 positioned on one of its generally flat surfaces. Lead wires 20 within the leads are coupled to proximal contacts 21, which are insertable into lead connectors 22 fixed in a header 23 on the IPG 10, which header can comprise an epoxy for example. Once inserted, the proximal contacts 21 connect to header contacts 24 within the lead connectors 22, which are in turn coupled by feedthrough pins 25 through a case feedthrough 26 to stimulation circuitry 28 within the case 12, which stimulation circuitry 28 is described below.

In the illustrated IPG 10, there are thirty-two electrodes (E1-E32), split between four percutaneous leads 15, or contained on a single paddle lead 19, and thus the header 23 may include a 2x2 array of eight-electrode lead connectors 22. However, the type and number of leads, and the number of electrodes, in an IPG is application specific and therefore can vary. The conductive case 12 can also comprise an electrode (Ec), and thus the electrode array 17 can include one or more leads and the case electrode 12. In a SCS application, the electrode lead(s) are typically implanted in the spinal column proximate to the dura in a patient's spinal cord, preferably spanning left and right of the patient's spinal column. The proximal contacts 21 are then tunneled through the patient's tissue to a distant location such as the buttocks where the IPG case 12 is implanted, where they are coupled to the lead connectors 22. In other IPG examples designed for implantation directly at a site requiring stimulation, the IPG can be lead-less, having electrodes 16 instead appearing on the body of the IPG 10 for contacting the patient's tissue. The IPG lead(s) can be integrated with and permanently connected to the IPG 10 in other solutions. The goal of SCS therapy is to provide electrical stimulation from the electrodes 16 to alleviate a patient's symptoms, such as chronic back pain.

IPG 10 can include an antenna 27a allowing it to communicate bi-directionally with a number of external devices discussed subsequently. Antenna 27a as shown comprises a conductive coil within the case 12, although the coil antenna 27a can also appear in the header 23. When antenna 27a is configured as a coil, communication with external devices preferably occurs using near-field magnetic induction. IPG 10 may also include a RadioFrequency (RF) antenna 27b. RF antenna 27b is shown within the header 23, but it may also be within the case 12. RF antenna 27b may comprise a patch, slot, or wire, and may operate as a monopole or dipole. RF antenna 27b preferably communicates using far-field electromagnetic waves, and may operate in accordance with any number of known RF communication standards, such as Bluetooth, Zigbee, MICS, and the like. The IPG 10 can also include an accelerometer 31 able to detect the orientation of the IPG 10 in the patient, which can be useful to determining a patient's posture (e.g., standing, prone, supine, etc.).

Stimulation in IPG 10 is typically provided by a sequence of waveforms (e.g., pulses) each of which may include a number of phases such as 30a and 30b, as shown in the example of Figure 2A. Stimulation parameters typically include amplitude (current A, although a voltage amplitude V can also be used); frequency (f); pulse width (PW) of the phases of the waveform such as 30a and 30b; the electrodes 16 selected to provide the stimulation; and the polarity of such selected electrodes, i.e., whether they act as anodes that source current to the tissue or cathodes that sink current from the tissue. These and possibly other stimulation parameters taken together comprise a stimulation program that the stimulation circuitry 28 in the IPG 10 can execute to provide therapeutic stimulation to a patient.

In the example of Figure 2A, electrode E1 has been selected as an anode (during first phase 30a), and thus sources a positive current of amplitude +A to the tissue. Electrode E2 has been selected as a cathode (again during first phases 30a), and thus sinks a corresponding negative current of amplitude -A from the tissue. However, more than one electrode may be selected to act as an anode at a given time, and more than one electrode may be selected to act as a cathode at a given time. The case electrode may also be selected as an anode or cathode by itself or along with one or more lead-based electrodes.

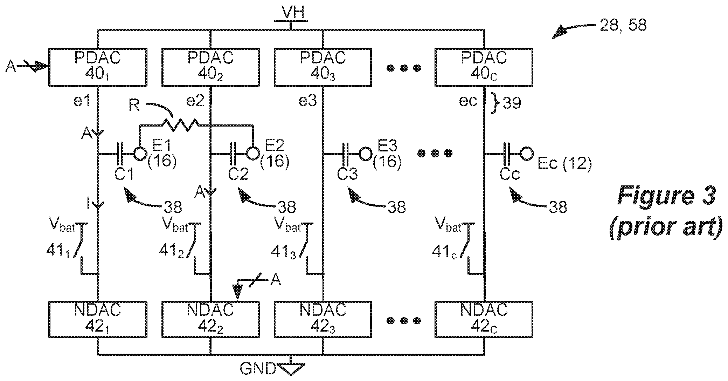

IPG 10 as mentioned includes stimulation circuitry 28 to form prescribed stimulation at a patient's tissue. Figure 3 shows an example of stimulation circuitry 28, which includes one or more current sources 40ᵢ and one or more current sinks 42ᵢ. The sources and sinks 40ᵢ and 42ᵢ can comprise Digital-to-Analog converters (DACs), and may be referred to as PDACs 40ᵢ and NDACs 42ᵢ in accordance with the Positive (sourced, anodic) and Negative (sunk, cathodic) currents they respectively issue. In the example shown, a NDAC/PDAC 40ᵢ/42ᵢ pair is dedicated (hardwired) to a particular electrode node ei 39. Each electrode node ei 39 is connected to an electrode Ei 16 via a DC-blocking capacitor Ci 38, for the reasons explained below. PDACs 40ᵢ and NDACs 42ᵢ can also comprise voltage sources.

Proper control of the PDACs 40ᵢ and NDACs 42ᵢ allows any of the electrodes 16 and the case electrode Ec 12 to act as anodes or cathodes to create a current through a patient's tissue, R, hopefully with good therapeutic effect. In the example shown, and consistent with the first phase 30a of Figure 2A, electrode E1 has been selected as an anode electrode to source current +A to the tissue R and electrode E2 has been selected as a cathode electrode to sink current -A from the tissue R. Thus PDAC 40₁ and NDAC 42₂ are activated and digitally programmed to produce the desired current, A, with the correct timing (e.g., in accordance with the prescribed frequency f and pulse width PW). Power for the stimulation circuitry 28 is provided by a compliance voltage VH, as described in further detail in U.S. Patent Application Publication 2013/0289665.

Other stimulation circuitries 28 can also be used in the IPG 10. In an example not shown, a switching matrix can intervene between the one or more PDACs 40; and the electrode nodes ei 39, and between the one or more NDACs 42ᵢ and the electrode nodes. Switching matrices allows one or more of the PDACs or one or more of the NDACs to be connected to one or more electrode nodes at a given time. Various examples of stimulation circuitries can be found in USPs 6,181,969, 8,606,362, 8,620,436, U.S. Patent Application Publications 2018/0071520 and 2019/0083796.

Much of the stimulation circuitry 28 of Figure 3, including the PDACs 40ᵢ and NDACs 42ᵢ, the switch matrices (if present), and the electrode nodes ei 39 can be integrated on one or more Application Specific Integrated Circuits (ASICs), as described in U.S. Patent Application Publications 2012/0095529, 2012/0092031, 2012/0095519, 2018/0071516, and 2018/0071513. As explained in these references, ASIC(s) may also contain other circuitry useful in the IPG 10, such as telemetry circuitry (for interfacing off chip with telemetry antennas 27a and/or 27b), circuitry for generating the compliance voltage VH, various measurement circuits, etc.

Also shown in Figure 3 are DC-blocking capacitors Ci 38 placed in series in the electrode current paths between each of the electrode nodes ei 39 and the electrodes Ei 16 (including the case electrode Ec 12). The DC-blocking capacitors 38 act as a safety measure to prevent DC current injection into the patient, as could occur for example if there is a circuit fault in the stimulation circuitry 28. The DC-blocking capacitors 38 are typically provided off-chip (off of the ASIC(s)), and instead may be provided in or on a circuit board in the IPG 10 used to integrate its various components, as explained in U.S. Patent Application Publication 2015/0157861.

Referring again to Figure 2A, the stimulation waveforms as shown are biphasic, with each waveform comprising a first phase 30a followed thereafter by a second phase 30b of opposite polarity. (Although not shown, an interphase period during which no active current is driven may intervene between the phases 30a and 30b). Both of the phases 30a and 30b are actively driven by the stimulation circuitry 28 by causing relevant PDACs 40ᵢ and NDACs 42ᵢ to drive the prescribed currents. Biphasic waveforms are useful to actively recover any charge that might be stored on capacitive elements in the current path, such as on the DC-blocking capacitors 38. To recover all charge by the end of the second phase 30b of each waveform (Vc1 = Vc2 = 0V), the first and second phases 30a and 30b are charged balanced at each electrode, with the first phase 30a providing a charge of +Q (+A * PW) and the second phase 30b providing a charge of -Q (-A * PW) at electrode E1, and with the first phase 30a providing a charge of -Q and the second phase 30b providing a charge of +Q at the electrode E2. In the example shown, such charge balancing is achieved by using the same phase width (PW) and the same amplitude (|A|) for each of the opposite-polarity phases 30a and 30b. However, the phases 30a and 30b may also be charged balance at each electrode if the product of the amplitude and pulse width of the two phases 30a and 30b are equal, or if the area under each of the phases (their integrals) is equal, as is known. Although not shown, the waveforms may also be monophasic, meaning that there is only one active phase, i.e., only first phase 30a or second phase 30b.

Figure 3 shows that stimulation circuitry 28 can include passive recovery circuitry, which is described further in U.S. Patent Application Publications 2018/0071527 and 2018/0140831. Specifically, passive recovery switches 41ᵢ may be attached to each of the electrode nodes ei 39, and are used to passively recover any charge remaining on the DC-blocking capacitors Ci 38 after issuance of a last pulse phase-i.e., after the second phase 30b if a biphasic pulses are used, or after the sole pulse phase if monophasic pulses are used. Note that passive charge recovery is illustrated as small exponentially-decaying curves during 30c in Figure 2A due to the R-C nature of the circuit, and this current may be positive or negative depending on whether phase 30a or 30b has a predominance of charge at a given electrode. These exponentially-decaying curves would be larger were monophasic pulses used.

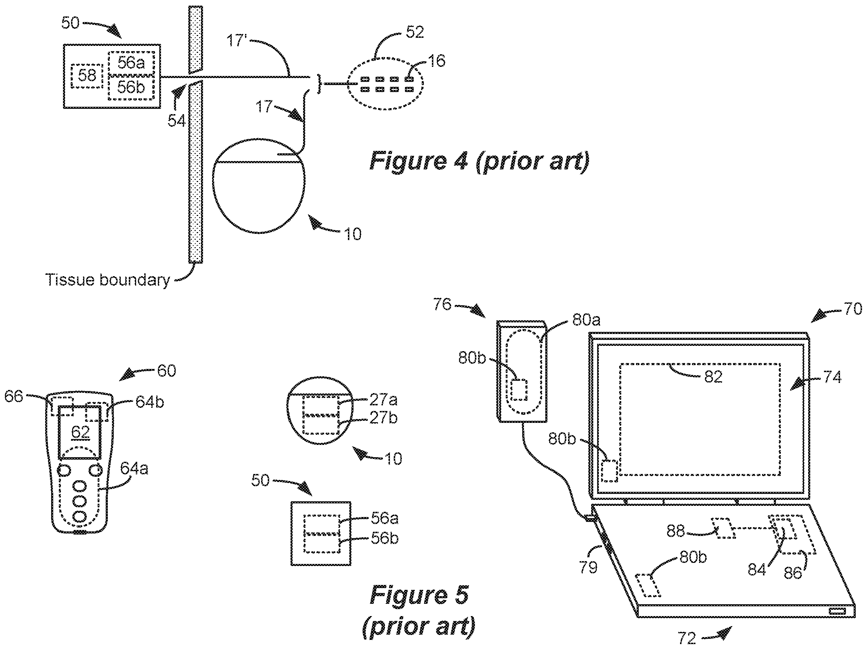

Figure 4 shows an external trial stimulation environment that may precede implantation of an IPG 10 in a patient. During external trial stimulation, stimulation can be tried on a prospective implant patient without going so far as to implant the IPG 10. Instead, one or more trial electrode arrays 17' (e.g., one or more trial percutaneous leads 15 or trial paddle leads 19) are implanted in the patient's tissue at a target location 52, such as within the spinal column as explained earlier. The proximal ends of the trial electrode array(s) 17' exit an incision 54 and are connected to an External Trial Stimulator (ETS) 50. The ETS 50 generally mimics operation of the IPG 10, and thus can provide stimulation to the patient's tissue via its stimulation circuitry 58, which may be equivalent or identical to stimulation circuitry 28 in the IPG 10. The ETS 50 is generally worn externally by the patient for a short while (e.g., two weeks), which allows the patient and his clinician to experiment with different stimulation parameters to hopefully find a stimulation program that alleviates the patient's symptoms (e.g., pain). If external trial stimulation proves successful, the trial electrode array(s) 17' are explanted, and a full IPG 10 and a permanent electrode array 17 (e.g., one or more percutaneous 15 or paddle 19 leads) are implanted as described above; if unsuccessful, the trial electrode array(s) 17' are simply explanted. Like the IPG 10, the ETS 50 can include one or more antennas to enable bi-directional communications with external devices such as those shown in Figure 5. Such antennas can include a near-field magnetic-induction coil antenna 56a, and/or a far-field RF antenna 56b, as described earlier. ETS 50 may also include a battery (not shown) for operational power.

Figure 5 shows various external devices that can wirelessly communicate data with the IPG 10 and the ETS 50, including a patient hand-held external controller 60, and a clinician programmer 70. Both of devices 60 and 70 can be used to wirelessly transmit a stimulation program to the IPG 10 or ETS 50-that is, to program their stimulation circuitries 28 and 58 to produce stimulation with a desired amplitude and timing, and at selected electrodes. Both devices 60 and 70 may also be used to adjust one or more stimulation parameters of a stimulation program that the IPG 10 or ETS 50 is currently executing. Devices 60 and 70 may also wirelessly receive information from the IPG 10 or ETS 50, such as various status information, etc.

External controller 60 can be as described in U.S. Patent Application Publication 2015/0080982 for example, and may comprise a controller dedicated to work with the IPG 10 or ETS 50. External controller 60 may also comprise a general purpose mobile electronics device such as a mobile phone which has been programmed with a Medical Device Application (MDA) allowing it to work as a wireless controller for the IPG 10 or ETS 50, as described in U.S. Patent Application Publication 2015/0231402. External controller 60 includes a Graphical User Interface (GUI), preferably including means for entering commands (e.g., buttons or selectable graphical icons) and a display 62, thus allowing the patient the ability to control the IPG 10 or ETS 50. The external controller 60's GUI enables a patient to adjust stimulation parameters, although it may have limited functionality when compared to the more-powerful clinician programmer 70, described shortly. The external controller 60 can have one or more antennas capable of communicating with the IPG 10 and ETS 50. For example, the external controller 60 can have a near-field magnetic-induction coil antenna 64a capable of wirelessly communicating with the coil antenna 27a or 56a in the IPG 10 or ETS 50. The external controller 60 can also have a far-field RF antenna 64b capable of wirelessly communicating with the RF antenna 27b or 56b in the IPG 10 or ETS 50.

Clinician programmer 70 is described further in U.S. Patent Application Publication 2015/0360038, and can comprise a computing device 72, such as a desktop, laptop, or notebook computer, a tablet, a mobile smart phone, a Personal Data Assistant (PDA)-type mobile computing device, etc. In Figure 5, computing device 72 is shown as a laptop computer that includes typical computer user interface means such as a screen 74, a mouse, a keyboard, speakers, a stylus, a printer, etc., not all of which are shown for convenience. Also shown in Figure 5 are accessory devices for the clinician programmer 70 that are usually specific to its operation as a stimulation controller, such as a communication "wand" 76 coupleable to suitable ports on the computing device 72, such as USB ports 79 for example.

The antenna used in the clinician programmer 70 to communicate with the IPG 10 or ETS 50 can depend on the type of antennas included in those devices. If the patient's IPG 10 or ETS 50 includes a coil antenna 27a or 56a, wand 76 can likewise include a coil antenna 80a to establish near-field magnetic-induction communications at small distances. In this instance, the wand 76 may be affixed in close proximity to the patient, such as by placing the wand 76 in a belt or holster wearable by the patient and proximate to the patient's IPG 10 or ETS 50. If the IPG 10 or ETS 50 includes an RF antenna 27b or 56b, the wand 76, the computing device 72, or both, can likewise include an RF antenna 80b to establish communication with the IPG 10 or ETS 50 at larger distances. The clinician programmer 70 can also communicate with other devices and networks, such as the Internet, either wirelessly or via a wired link provided at an Ethernet or network port.

To program stimulation programs or parameters for the IPG 10 or ETS 50, the clinician interfaces with a clinician programmer GUI 82 provided on the display 74 of the computing device 72. As one skilled in the art understands, the GUI 82 can be rendered by execution of clinician programmer software 84 stored in the computing device 72, which software may be stored in the device's non-volatile memory 86. Execution of the clinician programmer software 84 in the computing device 72 can be facilitated by controller circuitry 88 such as one or more microprocessors, microcomputers, FPGAs, DSPs, other digital logic structures, etc., which are capable of executing programs in a computing device, and which may comprise their own memories. In one example, controller circuitry 88 may comprise an i5 processor manufactured by Intel Corp., as described at https:// www.intel.com/ content/ www/ us/ en/ products/ processors/ core/ i5-processors.html. Such controller circuitry 88, in addition to executing the clinician programmer software 84 and rendering the GUI 82, can also enable communications via antennas 80a or 80b to communicate stimulation parameters chosen through the GUI 82 to the patient's IPG 10 or ETS 50.

The GUI of the external controller 60 may provide similar functionality because the external controller 60 can include the same or similar hardware and software programming as the clinician programmer 70. For example, the external controller 60 includes control circuitry 66 similar to the controller circuitry 88 in the clinician programmer 70, and may similarly be programmed with external controller software stored in device memory.

WO2006/107848 discloses an apparatus for detecting lead migration through the use of measured data about the tissue in the vicinity of the lead.

### SUMMARY

The invention is defined by the independent claims. A method is disclosed for adjusting a position of a pole configuration in an electrode array of an implantable stimulation device, which may comprise: (a) providing stimulation to the patient's tissue using the pole configuration at a position in the electrode array; (b) measuring a neural response from the pole configuration at the position at one or more sensing electrodes in the electrode array as a measured response; (c) comparing the measured response at each of the at least one sensing electrodes to a baseline response at a corresponding one of the sensing electrodes; and (d) if the measured response does not equal the baseline response, adjusting the position of the pole configuration in the electrode array and repeating steps (a)-(c) until the measured response equals or is closer to the baseline response.

In one example, in step (d) the position of the pole configuration is adjusted until the measured response equals the baseline response. In one example, in step (c) at least one feature of the measured response at each of the sensing electrodes is compared to the at least one feature of the baseline response at the corresponding one of the sensing electrodes. In one example, the at least one feature comprises a time or speed at which the measured response and the baseline response arrives at each of the sensing electrodes. In one example, the at least one feature comprises a duration of the measured response and the baseline response at each of the sensing electrodes. In one example, the at least one feature comprises an amplitude of the measured response and the baseline response at each of the sensing electrodes. In one example, prior to step (a), determining the baseline response by providing stimulation to the patient's tissue using the pole configuration at an initial position in the electrode array; measuring a neural response from the pole configuration at the initial position at the one or more sensing electrodes in the electrode array; and storing at least one feature of the measured neural response as received at each of the one or more sensing electrodes as the baseline response. In one example, in step (a) the stimulation is first provided using the pole configuration at the initial position. In one example, the method further comprises receiving an indication of a symptom of a patient at an external device in communication with the implantable stimulation device, wherein the method is automatically initiated if the indication is not suitable relative to a threshold. In one example, the method further comprises prior to step (a) determining a posture of the patient, wherein the baseline response at the one or more sensing electrodes corresponds to the determined posture of the patient. In one example, in step (d) the measured response and the baseline response are used to determine a direction for adjusting the position of the pole configuration. In one example, in step (d) the measured response and the baseline response are further used to determine a distance for adjusting the position of the pole configuration in the direction. In one example, step (d) further comprises adjusting other stimulation parameters of the pole configuration that do not affect the position of the pole configuration. In one example, there are a plurality of sensing electrodes. In one example, the sensing electrodes are aligned rostral-caudally in the electrode array. In one example, the sensing electrodes are aligned medio-laterally in the electrode array.

A system is disclosed, which may comprise: an implantable stimulation device comprising an electrode array configured to provide stimulation to a patient's tissue; an external device configured to communicate with the implantable stimulation device; and an algorithm configured to operate at least in part in the implantable stimulation device, wherein the algorithm is configured to (a) provide stimulation to the patient's tissue using the pole configuration at a position in the electrode array; (b) measure a neural response from the pole configuration at the position at one or more sensing electrodes in the electrode array as a measured response; (c) compare the measured response at each of the at least one sensing electrodes to a baseline response at a corresponding one of the sensing electrodes; and (d) if the measured response does not equal the baseline response, adjust the position of the pole configuration in the electrode array and repeating steps (a)-(c) until the measured response equals or is closer to the baseline response.

In one example, the algorithm is configured to operate wholly in the implantable stimulation device. In one example, steps (a) and (b) are configured to operate in the implantable stimulation device, and wherein steps (c) and (d) are configured to operate in the external device. In one example, in step (d) the position of the pole configuration is adjusted until the measured response equals the baseline response. In one example, in step (c) at least one feature of the measured response at each of the sensing electrodes is compared to the at least one feature of the baseline response at the corresponding one of the sensing electrodes. In one example, the at least one feature comprises a time or speed at which the measured response and the baseline response arrives at each of the sensing electrodes. In one example, the at least one feature comprises a duration of the measured response and the baseline response at each of the sensing electrodes. In one example, the at least one feature comprises an amplitude of the measured response and the baseline response at each of the sensing electrodes. In one example, the baseline response is stored in a database, wherein the baseline response comprises a neural response measured at the one or more sensing electrodes in the electrode array in response to providing stimulation using the pole configuration at an initial position in the electrode array. In one example, the algorithm starts in step (a) by providing stimulation to the patient's tissue using the pole configuration at the initial position in the electrode array. In one example, the database is stored in the implantable stimulation device. In one example, the database is stored in the external device. In one example, the database comprises a neural response measured at the one or more sensing electrodes at a plurality of different postures of the patient. In one example, the implantable stimulation device includes a means for determining a posture of the patient. In one example, in step (c) the measured response at each of the at least one sensing electrodes is compared to a baseline response at a corresponding one of the sensing electrodes as stored for the determined posture. In one example, the external device is configured to provide a Graphical User Interface (GUI), and wherein the GUI is configured to receive an indication of a symptom of a patient. In one example, the algorithm is automatically initiated if the indication is not suitable relative to a threshold. In one example, in step (d) the measured response and the baseline response are used to determine a direction for adjusting the position of the pole configuration. In one example, in step (d) the measured response and the baseline response are further used to determine a distance for adjusting the position of the pole configuration in the direction. In one example, step (d) further comprises adjusting other stimulation parameters of the pole configuration that do not affect the position of the pole configuration. In one example, the algorithm is configured to choose the one or more sensing electrodes. In one example, there are a plurality of sensing electrodes. In one example, the sensing electrodes are aligned rostral-caudally in the electrode array. In one example, the sensing electrodes are aligned medio-laterally in the electrode array.

A method is disclosed for operating an implantable stimulation device having an electrode array, which method may comprise: (a) providing stimulation to the patient's tissue using the pole configuration at a position in the electrode array; (b) measuring a neural response from the pole configuration at the position at one or more sensing electrodes in the electrode array as a measured response; (c) assessing the consistency of the measured response at each of the at least one sensing electrodes to a baseline response at a corresponding one of the sensing electrodes; (d) using the assessed consistency as determined in step (c) to determine whether to: (i) adjust a position of the pole configuration in the electrode array, or (ii) adjust other stimulation parameters of the pole configuration that do not affect the position of the pole configuration; and (e) providing the adjustment of (i) or (ii) depending on the determination of step (d).

In one example, the method may further comprise: in step (d), using the assessed consistency as determined in step (c) to determine whether to: (iii) select one or more new sensing electrodes in the electrode array; and in step (e), providing the adjustment of (i) or (ii), or the selection of (iii), depending on the determination of step (d). The method may also include any of the other concepts described above.

A system is disclosed, which may comprise: an implantable stimulation device comprising an electrode array configured to provide stimulation to a patient's tissue; an external device configured to communicate with the implantable stimulation device; and an algorithm configured to operate at least in part in the implantable stimulation device, wherein the algorithm is configured to (a) provide stimulation to the patient's tissue using the pole configuration at a position in the electrode array; (b) measure a neural response from the pole configuration at the position at one or more sensing electrodes in the electrode array as a measured response; (c) assess the consistency of the measured response at each of the at least one sensing electrodes to a baseline response at a corresponding one of the sensing electrodes; (d) use the assessed consistency as determined in step (c) to determine whether to: (i) adjust a position of the pole configuration in the electrode array, or (ii) adjust other stimulation parameters of the pole configuration that do not affect the position of the pole configuration; and (e) provide the adjustment of (i) or (ii) depending on the determination of step (d).

In one example, the algorithm is further configured to: in step (d), use the assessed consistency as determined in step (c) to determine whether to: (iii) select one or more new sensing electrodes in the electrode array; and in step (e), provide the adjustment of (i) or (ii), or the selection of (iii), depending on the determination of step (d). The system may also include any of the other concepts described above.

### BRIEF DESCRIPTION OF THE DRAWINGS

Figure 1 shows an Implantable Pulse Generator (IPG), in accordance with the prior art.
Figures 2A and 2B show an example of stimulation waveforms producible by the IPG or in an External Trial Stimulator (ETS), in accordance with the prior art.
Figure 3 shows stimulation circuitry useable in the IPG or ETS, in accordance with the prior art.
Figure 4 shows an ETS environment useable to provide stimulation before implantation of an IPG, in accordance with the prior art.
Figure 5 shows various external devices capable of communicating with and programming stimulation in an IPG and ETS, in accordance with the prior art.
Figure 6 shows circuitry in an IPG or ETS for providing simulation to an electrode array and for sensing neural responses such as Evoked Compound Action Potentials (ECAPs) using the electrode array.
Figures 7A and 7B show movement of an electrode array in a patient's tissue causing a stimulating pole configuration to become misaligned with a stimulation target in the tissue.
Figure 8 shows a Graphical User Interface (GUI) for establishing a pole configuration in an electrode array, and shows use of an electrode configuration algorithm to establish poles in the configuration at prescribed positions.
Figure 9 shows an ECAP baseline database which stores baseline ECAP measurements for a given pole configuration, and for a number of patient postures.
Figures 10A-10C show examples of ECAP measurements at sensing electrodes, and how such measurements can differ from ECAP baselines when the electrode array moves in the tissue.
Figure 11 shows a therapy adjustment algorithm which compares ECAP measurements to the ECAP baselines to, among other details, move the position of the pole configuration to cause the ECAP measurements to equal, or at least come closer to, the ECAP baselines.
Figures 12A and 12B show further details of the algorithm, and specifically steps that can be used to adjust the position of the pole configuration.
Figure 13 shows how the pole configuration position can be adjusted in a rostral-caudal direction by the algorithm in an informed manner by comparison of the measured and baseline ECAPs.
Figures 14A and 14B show how the pole configuration position can be adjusted in a medio-lateral direction by the algorithm in an informed manner by comparison of the measured and baseline ECAPs.
Figure 15A and 15B show how the pole configuration position can be adjusted when the stimulating and sensing electrodes are on different leads.

### DETAILED DESCRIPTION

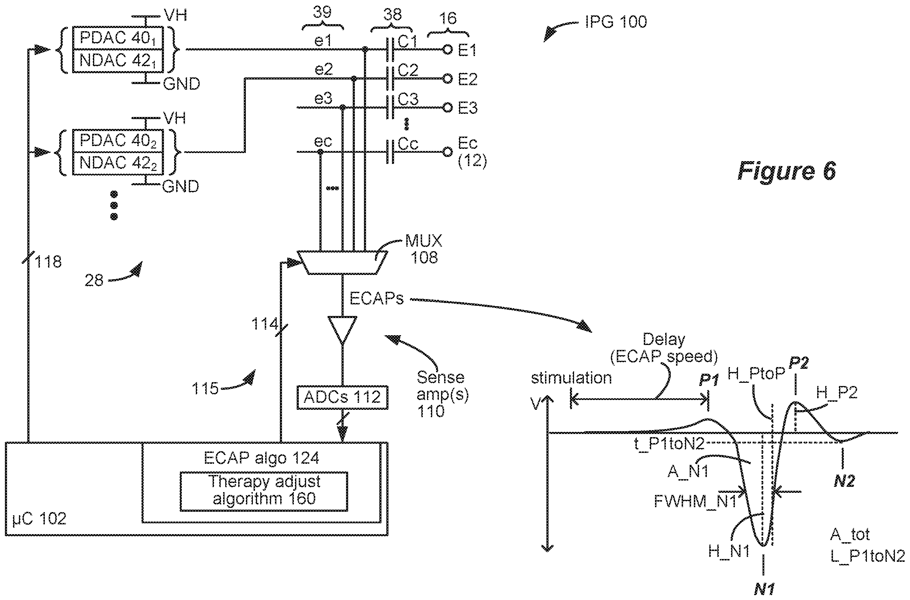

An increasingly interesting development in pulse generator systems, and in Spinal Cord Stimulator (SCS) pulse generator systems specifically, is the addition of sensing capability to complement the stimulation that such systems provide. For example, and as explained in U.S. Patent Application Publication 2017/0296823, it can be beneficial to sense a neural response in neural tissue that has received stimulation from an SCS pulse generator. One such neural response is an Evoked Compound Action Potential (ECAP). An ECAP comprises a cumulative response provided by neural fibers that are recruited by the stimulation, and essentially comprises the sum of the action potentials of recruited fibers when they "fire." An ECAP is shown in Figure 6, and comprises a number of peaks that are conventionally labeled with P for positive peaks and N for negative peaks, with P1 comprising a first positive peak, N1 a first negative peak, P2 a second positive peak and so on. Note that not all ECAPs will have the exact shape and number of peaks as illustrated in Figure 6, because an ECAP's shape is a function of the number and types of neural fibers that are recruited and that are involved in its conduction. An ECAP is generally a small signal, and may have a peak-to-peak amplitude on the order of tens of microVolts to tens of milliVolts.

Also shown in Figure 6 is circuitry for an IPG 100 (or an ETS) that is capable of providing stimulation and sensing a resulting ECAP or other neural response or signal. The IPG 100 includes control circuitry 102, which may comprise a microcontroller for example such as Part Number MSP430, manufactured by Texas Instruments, which is described in data sheets at http://www.ti.com/ lsds/ ti/ microcontroller/ 16-bit_msp430/ overview.page? DCMP = MCU_other& HQS = msp430. Other types of controller circuitry may be used in lieu of a microcontroller as well, such as microprocessors, FPGAs, DSPs, or combinations of these, etc. Control circuitry 102 may also be formed in whole or in part in one or more Application Specific Integrated Circuits (ASICs), such as those described earlier.

The IPG 100 also includes stimulation circuitry 28 to produce stimulation at the electrodes 16, which may comprise the stimulation circuitry 28 shown earlier (Fig. 3). A bus 118 provides digital control signals from the control circuitry 102 (and possibly from an ECAP algorithm 124, described below) to one or more PDACs 40ᵢ or NDACs 42ᵢ to produce currents or voltages of prescribed amplitudes (A) for the stimulation pulses, and with the correct timing (PW, f). As noted earlier, but not shown in Figure 6, a switch matrices could intervene between the PDACs and the electrode nodes 39, and between the NDACs and the electrode nodes, to route their outputs to one or more of the electrodes, including the conductive case electrode 12 (Ec). Control signals for switch matrices, if present, may also be carried by bus 118. Notice that the current paths to the electrodes 16 include the DC-blocking capacitors 38 described earlier, which provide safety by preventing the inadvertent supply of DC current to an electrode and to a patient's tissue. Passive recovery switches 41ᵢ (Fig. 3) could also be present, but are not shown in Figure 4 for simplicity.

IPG 100 also includes sensing circuitry 115, and one or more of the electrodes 16 can be used to sense neural responses such as the ECAPs described earlier. In this regard, each electrode node 39 is further coupleable to a sense amp circuit 110. Under control by bus 114, a multiplexer 108 can select one or more electrodes to operate as sensing electrodes by coupling the electrode(s) to the sense amps circuit 110 at a given time, as explained further below. Although only one multiplexer 108 and sense amp circuit 110 is shown in Figure 6, there could be more than one. For example, there can be four multiplexer 108/sense amp circuit 110 pairs each operable within one of four timing channels supported by the IPG 100 to provide stimulation. The analog waveform comprising the ECAP is preferably converted to digital signals by one or more Analog-to-Digital converters (ADC(s)) 112, which may sample the waveform at 50 kHz for example. The ADC(s) 112 may also reside within the control circuitry 102, particularly if the control circuitry 102 has A/D inputs.

As shown, an ECAP algorithm 124 is programmed into the control circuitry 102 to receive and analyze the digitized ECAPs. One skilled in the art will understand that the ECAP algorithm 124 can comprise instructions that can be stored on non-transitory machine-readable media, such as magnetic, optical, or solid-state memories within the IPG 100 (e.g., stored in association with control circuitry 102).

In the example shown in Figure 6, the ECAP algorithm 124 operates within the IPG 100 to determine one or more ECAP features, which may include but are not limited to:
- a height of any peak (e.g., H_N1) present in the ECAP;
- a peak-to-peak height between any two peaks (such as H_PtoP from N1 to P2);
- a ratio of peak heights (e.g., H_N1 / H_P2);
- a peak width of any peak (e.g., the full width half maximum of a N1, FWHM_N1);
- an area under any peak (e.g., A_N1);
- a total area (A_tot) comprising the area under positive peaks with the area under negative peaks subtracted or added;
- a length of any portion of the curve of the ECAP (e.g., the length of the curve from P1 to N2, L_P1toN2)
- any time defining the duration of at least a portion of the ECAP (e.g., the time from P1 to N2, t_P1toN2);
- a time delay from stimulation to issuance of the ECAP, which is indicative of the neural conduction speed of the ECAP, which can be different in different types of neural tissues;
- non-time domain measurements such as frequency analysis in the Fourier domain, or wavelet analysis more generally;
- any mathematical combination or function of these variables (e.g., H_N1 / FWHM_N1 would generally specify a quality factor of peak N1).

Once the ECAP algorithm 124 determines one or more of these features, it may then adjust the stimulation that the IPG 100 provides, for example by providing new data to the stimulation circuitry 28 via bus 118. This is explained further in U.S. Patent Application Publications 2017/0296823 and 2019/0099602. In one simple example, the ECAP algorithm 124 can review the height of the ECAP (e.g., its peak-to-peak voltage), and in closed loop fashion adjust the amplitude I of the stimulation current to try and maintain the ECAP to a desired value.

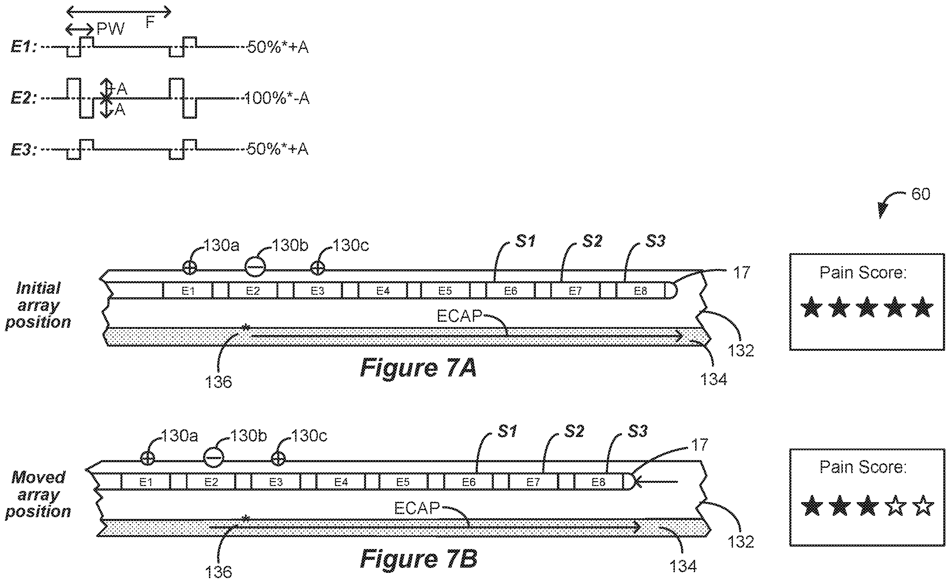

Figures 7A and 7B illustrate movement of the electrode array 17 within a patient, with Figure 7A showing an initial position and Figure 7B a moved position. (Electrode array 17 can include the array 17' from an ETS 50 as well as shown in Figure 4, but from this point discussion assumes use of an IPG with its array 17. The disclosed technique however can however be used with an ETS and its electrode array 17' as well). The electrode arrays 17 are shown in cross section as positioned in the epidural space 132 of the spinal column proximate to the dura 134. In the initial position of Figure 7A, a stimulation program has been determined for the patient that is effective to recruit a stimulation target in the spinal column, represented generically as element 136. In this example, the stimulation program defines a particular pole configuration, which in this example comprises a tripole, with a central cathode pole 130b and two flanking anodes 130a and 130c. The tripole is formed in this example with the cathode pole 130b at electrode E2, which receives 100% of a specified current A output by the stimulation circuitry 28 as a cathodic current (100% * -A). The anode poles 130a and 130c are formed at electrodes E1 and E3, which share the specified current equally as an anodic current (50% * +A). Use of a tripole is merely one example, and other pole configurations (e.g., monopoles, bipoles, etc.) could be used as well.

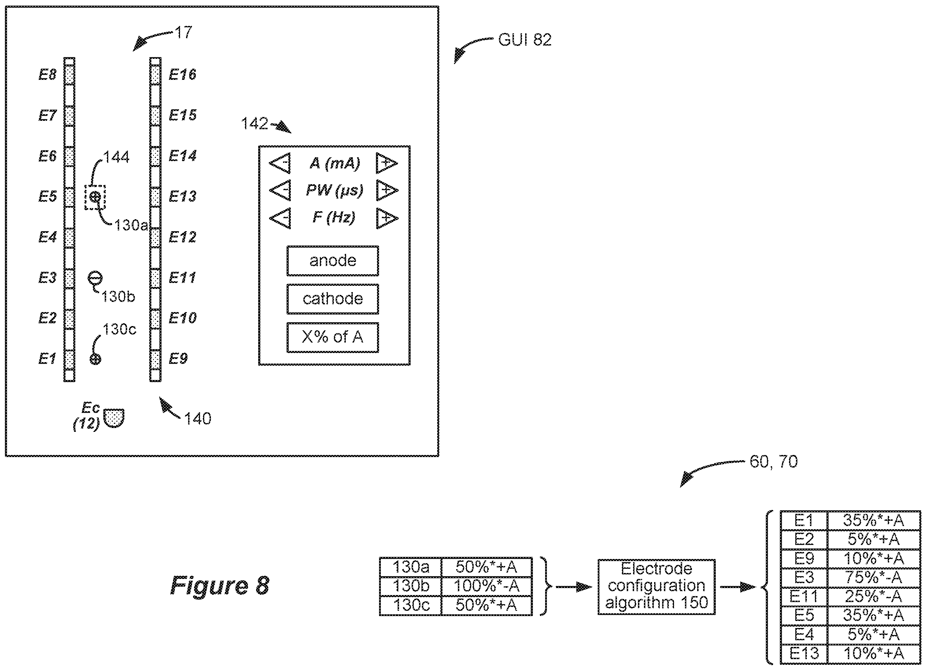

The poles 130 in a pole configuration do not need to be positioned at the physical position of the electrodes as shown in Figures 7A and 7B. Instead, the poles 130 can be positioned at any random position in the electrode array 17. This is shown in Figure 8, which additionally shows the GUI 82 on the external device (e.g., the clinician programmer 70 or external controller 60, Fig. 5) used to program the IPG. The GUI 82 can include a leads interface 140 showing a depiction of the electrode array 17, perhaps with reference to its location within the patient (e.g., with reference to various vertebrae). The GUI 82 can further include a parameters interface 142 used to set various stimulation parameters, such as the amplitude (A), pulse width (PW), and frequency (F) of the stimulation pulses. In reality the parameters interface 142 can be much more complicated, and can include many other options to define the stimulation to be provided. A cursor 144, controllable by a mouse or other computer peripheral, can be used to select particular electrodes 16 in the electrode array 17, or otherwise to set the position the poles 130 in the electrode array. A selected electrode or pole can be designated as an anode or cathode in the parameters interface 142, and a percentage X% of the current A that that electrode or pole is to receive can also be defined.

When the poles 130 in a pole configuration are not positioned at the physical position of the electrodes, an electrode configuration algorithm 150 operable in the external device 60 or 70 can compute what physical electrodes should be active, and with what polarities and current percentages, to best form the poles at the desired positions. The reader is assumed familiar with this electrode configuration algorithm 150, and it is described further for example in U.S. Patent Application Publication 2019/0175915. For example, assume in Figure 8 that cathode pole 130b is to receive 100% of the cathodic current (%100*-A), and anode poles 130a and 130c are each to receive 50% of the anodic current (50%*+A). Because the cathode pole 130b is closest to electrode E3 but also somewhat close to E11, the electrode configuration algorithm 150 may activate both of electrodes E3 and E11 in a manner to share the cathodic current (75%*-A and 25%*-A respectively) to in effect create cathode pole 130b as a virtual pole between E3 and E11. Likewise, anode pole 130a is close to E5, but also somewhat close to E13 and E4. As such, the electrode configuration algorithm 150 may share the anodic current allocated to anode pole 130a (50%*+A) by prescribing currents of 35%*+A, 10%*+A, and 5%*+A at E5, E13, and E4 respectively. Anode pole 130c may likewise be defined by the electrode configuration algorithm 150 by prescribing currents of 35%*+A, 10%*+A, and 5%*+A at electrodes E1, E9, and E2 respectively.

Returning to Figure 7B, it is seen that the electrode array 17 has moved (to the left) in the patient. This may occur due to a change in the patient's posture. For example, the patient may have moved from a sitting to a standing position, causing the electrode array to move slightly. The electrode array 17 may also have moved over time due to lead migration. In any event, the position of the poles 130 has now shifted relative to the stimulation target 136. As a result, the stimulation program may no longer adequately recruit this target 136, which may worsen the patient's pain symptoms. This reduction in effectiveness may be indicated by the patient. For example, the patient may enter a pain score in the graphical user interface of an external device such as his external controller 60 (Fig. 5). The patient may for example rate his pain using a "star rating" system, with five star signaling good pain relief, as in Figure 7A. In Figure 7B, where the electrode array has shifted and thus the pole configuration no longer targets the stimulation target 136 as it once did, it is seen that the patient is now experiencing more pain, providing only a three-star rating.

Also shown in Figures 7A and 7B is neural conduction of an ECAP as produced by the stimulation program. The ECAP generally travels by neural conduction both rostrally toward the brain and caudally away from the brain, although travel in only one direction is shown. The ECAP passes through neural tissue in the spinal cord with a speed which is dependent on the neural fibers involved in the conduction. In one example, the ECAP may move at a speed of about 5 cm / 1 ms.

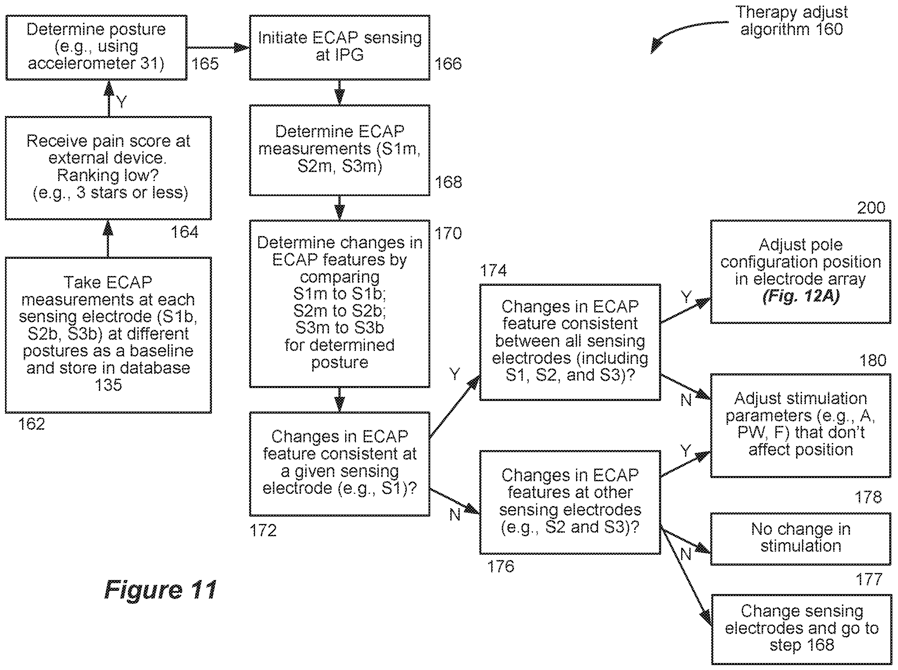

In this disclosure, ECAP sensing is used to infer that the electrode array 17 may have moved in the patient, with a therapy adjustment algorithm 160 (Fig. 11 et seq.) used to adjust the position of the pole configuration to compensate for such movement. The therapy adjustment algorithm 160 may comprise part of the ECAP algorithm 124, as shown in Figure 6, and may similarly comprise instructions that can be stored on non-transitory machine-readable media in the IPG.

In a preferred example of the technique, one or more sensing electrodes are selected (e.g., by multiplexer 108, Fig. 6) to sense an ECAP in response to the prescribed stimulation. Three such sensing electrodes-S1, S2, and S3-are illustrated in Figures 7A and 7B. The sensing electrodes Si may be selected by the user (e.g., using GUI 82 of an external device), or may be selected by the ECAP algorithm 124 or the therapy adjustment algorithm 160 in the IPG. Preferably, the sensing electrodes Si are selected at a logical distance from the stimulating electrodes. For example, in Figures 7A and 7B, where electrodes E1-E3 are used for stimulation, electrodes E6-E8 may be chosen as sensing electrodes S1-S3. Choosing sensing electrodes Si at a sensibly far distance from the stimulating electrodes is desired to make sure that stimulation artifacts (i.e., the electric field produced in the tissue due to the stimulation) are not too large at the sensing electrodes, which artifacts might otherwise mask the small-signal ECAP signals. However, the sensing electrodes should also be suitably close to the stimulating electrodes such that the ECAP, which has an amplitude that attenuates with distance, is still large enough to be reliably sensed.

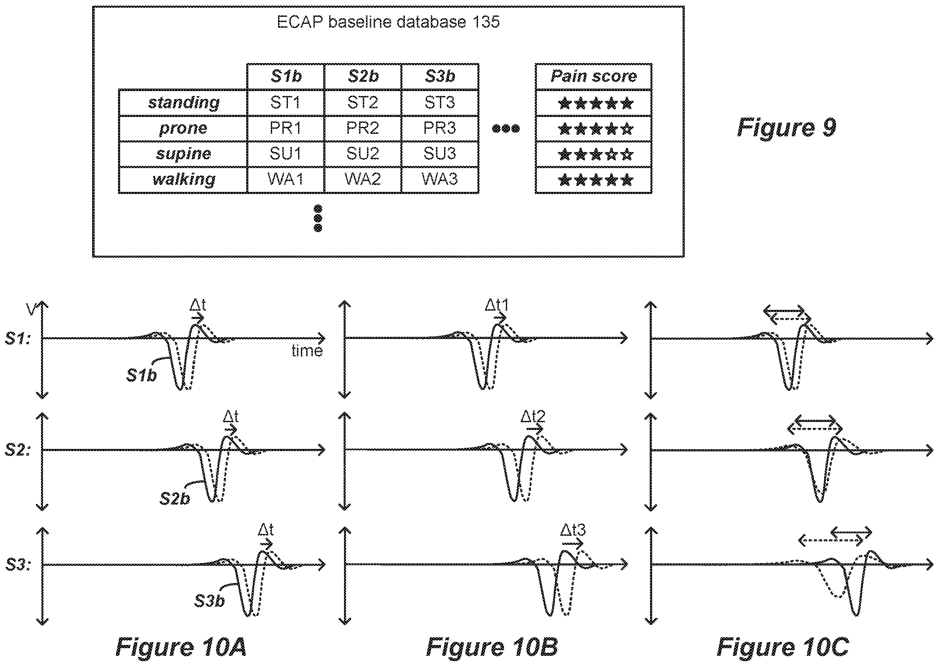

Figure 9 shows an ECAP baseline database 135 that can be used in conjunction with the therapy adjustment algorithm 160. Database 135 stores baseline information about sensed ECAPs that can be used to infer that the electrode array might have moved in the patient, as explained subsequently. The database 135 can be populated during a fitting session. For example, once an effective pole configuration has been determined for the patient (such as the tripole described earlier), ECAP baseline measurements can be taken at the selected sensing electrodes and stored in the database 135. The database 135 may also be populated, or updated, during operation of the therapy adjustment algorithm 160. The database 135 may be stored in the IPG, such as in conjunction with the therapy adjustment algorithm 160, or may also reside in an external device 60 or 70.

In Figure 9, baseline information is shown for sensing electrodes S1 (S1b), S2 (S2b) and S3 (S3b). The baseline information may comprise any information that is useful to understanding ECAPs as sensed in the patient before the electrode array 17 has moved. It may comprise the entire waveform of the ECAP as sensed at the electrodes, or one or more ECAP features described earlier (e.g., H_N1, H_PtoP, H_N1/H_P2, etc.).

Because patient posture can cause the electrode array 17 to move, ECAP baseline information is preferably stored in database 135 in association with particular postures. For example, database 135 can store ECAP baseline information at the various sensing electrodes when the patient is standing (S1b=ST1, S2b=ST2, S3b=ST3), prone (e.g., S1b=PR1), supine (e.g., S1b=SU1). ECAP baseline information can also be stored when a patient is engaging in a particular activity, such as walking (e.g., S1b=WA1). ("Posture" as used herein also includes patient activity for simplicity). Optionally, the ECAP baseline information may also be stored with a pain score. As explained later, this can inform the therapy adjustment algorithm 160 when it may be necessary to adjust the patient's therapy.

Figure 10A-10C shows ECAP baselines S1b-S3b as sensed at the sensing electrodes S1-S3 and stored when the electrode array 17 is in its initial position in Figure 7A (solid lines). In this example, the ECAP passes the sensing electrodes S1-S3 sequentially in accordance with the speed of neural conduction. In reality, the amplitude of the ECAP (e.g., its peak-to-peak height) would reduce at more distant sensing electrodes as the ECAP disperses in the neural tissue; for example, the magnitude of the ECAP as sensed at S3 would be smaller than at S 1. This however is not shown for convenience.

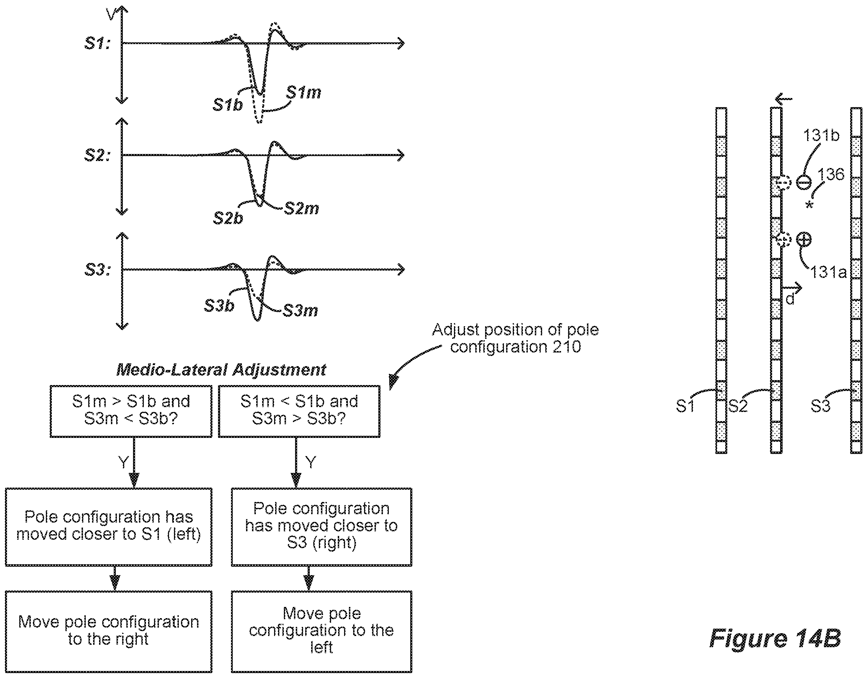

Figures 10A-10C additionally show manners in which sensed ECAPs can change when the electrode array 17 has moved from its initial position in Figure 7B (dotted lines). As these figures show, movement of the electrode array 17 can cause various changes in the sensed ECAPs, and such changes can result from different factors. For example, movement of the electrode array 17 may bring the electrodes closer to or farther from the neural tissue (e.g., the dura 134) in the spinal column. Movement will also more generally change the volume of neural tissue involved in the conduction of the ECAPs. In any event, such movement can manifest in different ways. In Figure 10A, the ECAPs do not change shape, but generally arrive at the sensing electrodes at a slightly later time, Δt. In Figure 10B, the ECAPs do not change shape, but generally arrive at the sensing electrodes at progressively longer times Δt1, Δt2, and Δt3, indicating at the neural conduction has become slower. In Figure 10C, the duration of the ECAP changes, becoming longer and more dispersed at more distant sensing electrodes (like S3). Movement of the electrode array can also cause the sensed ECAP to vary in amplitude (e.g., Fig. 14B). Other changes in the sensed ECAPs can result as the electrode array 17 moves, and Figures 10A-10C simply show some examples of this. The important point is changes in measured ECAPs from their baseline values Sib can indicate that the electrode array 17 might have moved in the tissue, and therefore that the prescribed therapy is no longer being provided to the optimal position. Accordingly, and as explained later, the position of pole configuration in the electrode array 17 can be moved using the therapy adjustment algorithm 160 to account for such movement and to adjust therapy to the proper position.

Figure 11 shows an example of therapy adjustment algorithm 160. Not all shown steps are necessary in useful embodiments, and other steps could be added. Further, while certain aspects of the algorithm 160 occur within the IPG (e.g., as part of ECAP algorithm 124), portions may also occur in conjunction with an external device 60 or 70 in communication with the IPG, as assisted by telemetry between the two devices. For example, the IPG may provide stimulation and measurement neural response such as ECAPs, and wirelessly telemeter the ECAP, or its features, to the external device. The external device may in turn compare the measures neural responses to baseline responses, and if necessary adjust the position of the pole configuration by sending appropriate control instructions to the IPG. Alternatively, the algorithm 160 may operate exclusively within the IPG.

Step 162 begins with population of the database 135 with baseline ECAP information, which again can occur at different patient postures, as explained previously with respect to Figure 9. At step 164, a patient enters a pain score indicative of his symptoms at an external device, such as his patient external controller 60. This can be a useful step in the algorithm 160, as operation of the algorithm 160 may not be necessary unless the patient's symptoms worsen. At step 164, operation of the algorithm 160 can continue if the patient's pain score drops, such as below a threshold (e.g., three stars or less). Optionally, the patient's posture (step 165) can also be determined at step 164 (using accelerometer 31 (Fig. 1) for example), with the baseline pain score for the relevant posture pulled from the database 135. This can allow the algorithm 160 to determine at step 164 if the patient's entered pain score is low relative to that baseline. For example, if the baseline pain score for a particular posture is three stars, and the patient enters three stars, the algorithm 160 may stop as the patient's condition hasn't worsened.

In any event, the algorithm 160 continues at step 165 by determining the patient's posture as just mentioned (if not determined already). This posture determination can be made in different manners, but in one example can involve querying information from the accelerometer 31 (Fig. 1) inside of the patient's IPG. Other means of determining a patient's posture in the IPG can be used as well, such as the technique disclosed in USP 9,446,243. At step 166, ECAP sensing is initiated, and at step 168, measured ECAPs S1m, S2m, and S3m are determined at the sensing electrodes. In step 170, the baseline ECAP information S1b, S2b, and S3b for the determined posture (step 165) is queried, and the measured information is compared to the baseline information. That is, S1m is compared to S1b, S2m is compared to S2b, and S3m is compared to S3b.

This comparison step 170 can occur in different manners, and can involve review of one or more ECAP features such as those described earlier. For example, the timing of arrival of the ECAP as gleaned from various peaks in the baseline and measured ECAPs can be compared similar to what was shown in Figures 10A and 10B, as can their amplitudes; the duration of the ECAPs can be compared, similar to what was shown in Figure 10C, etc.

It should be noted that therapy adjust algorithm 160 doesn't necessarily need to start upon receipt of a patient pain score at step 164. In another example, the IPG may be programmed to take periodic ECAP measurements, Sim. In this case, these measurements Sim can automatically be compared to the baseline measurements Sib (e.g., at step 170) for a determined posture to allow the algorithm 160 to decide if therapy adjustment might be indicated.

Steps 172-176 are example steps used to determine the type of therapy adjustment to be made, which can depend on the consistency between the various comparisons. These steps are not strictly required in a useful implementation, but are useful in determining whether it may be reasonable to not change therapy at all (step 178); to select new sensing electrodes (step 177); to change only the stimulation parameters (such as amplitude, pulse width or frequency) that do not affect the position of the pole configuration (step 180); or to change the position of the pole configuration 200 as may be necessary in the case of electrode array movement.

Step 172 inquires whether ECAP feature changes are consistent at a given sensing electrode, such as at S1. If not, step 176 can inquire whether ECAP feature changes are present at other sensing electrodes. If not, the measured ECAPs Sim vary too randomly when compared to the baseline ECAPs Sib, and it may then be unwarranted to change stimulation (step 178). Alternatively, because different sensing electrodes might provide a clearer and more consistent picture, new sensing electrodes can be chosen in step 177, and the process repeated by taking new ECAP measurements Sim at step 168. If at step 176 ECAP feature changes are present at other sensing electrodes, there may be enough consistency to warrant adjusting stimulation parameters (e.g., A, PW, F) that don't involve adjusting the position of the stimulation in the electrode array (step 180).

If at step 172 there are consistent ECAP features at a given sensing electrode, then step 174 can inquire whether such feature changes are consistent at the other sensing electrodes. If not, there may again be reason to adjust stimulation parameters, but not the position of the pole configuration (step 180). By contrast, if such feature changes are consistent at the other sensing electrodes-like the feature changes shown in Figures 10A-10C-this may suggest that the electrode array 17 has moved. Therefore, the algorithm 160 may determine that it is warranted to move the position of the pole configuration (step 200).

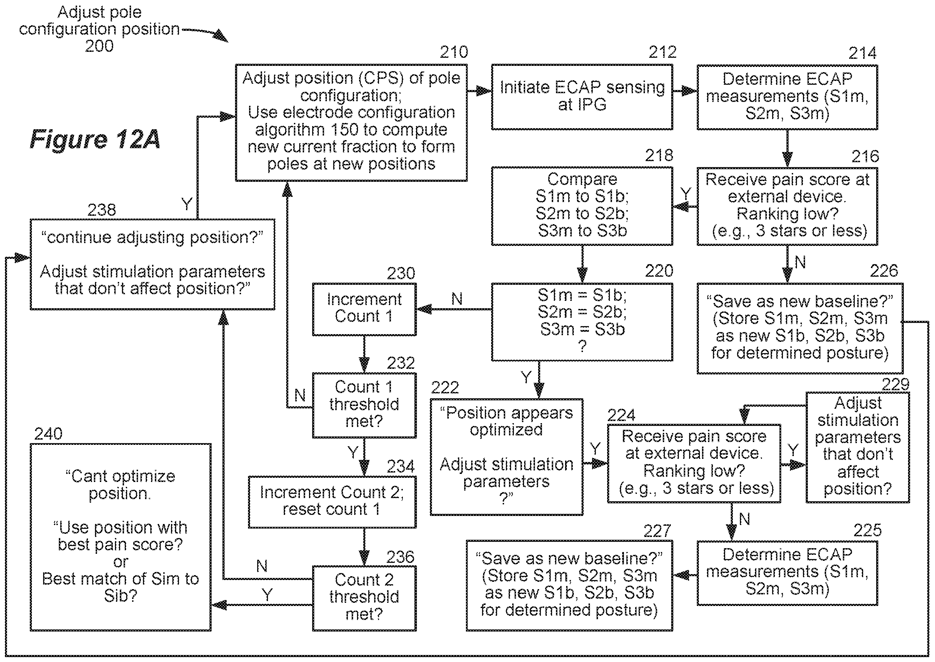

Further details regarding how the algorithm 160 can adjust the position of the pole configuration at step 200 are shown in Figure 12A. A goal of step 200 is to change the position of the pole configuration in the electrode array 17 until the measured ECAPs (Sim) equal the baseline ECAPs (Sib), or at least until Sim is brought closer to Sib. Thus, in a first step 210, the pole configuration is adjusted to a new position. Notice in this step that movement of the pole configuration can involve use of the electrode configuration algorithm 150 (Fig. 8) to determine which electrodes to activate, and with what polarities and current percentages.

In step 212 ECAP sensing is initiated, and in step 214 ECAP measurements S1m, S2m, and S3m are taken. At this point it may be reasonable to receive the patient's pain score again to gauge whether the position adjustment has been effective, in step 216. If the patient's pain score is no longer low, it may be reasonable to simply allow the patient to accept the position-adjusted therapy without need to compare the ECAP measurements Sim to the ECAP baselines Sib. Further, in step 226, because the patient's symptoms have improved, it may be reasonable to allow the patient to store the ECAP measurements Sim as the new baseline measurements Sib for the posture determined earlier (Fig. 11, step 165), and therefore the patient's external device may prompt the patient to this effect. Thus, the ECAP baselines Sib can be set to Sim in the database 135, and the pain score at step 216 can also be stored in the database for the determined posture as well.

At this point (step 226), even though the position-adjusted therapy has improved the patient's symptoms, it may be reasonable to continue adjusting the position; perhaps the patient's symptoms can be further improved, and an even better pain score received. Therefore, the algorithm 160 may continue to step 238, which prompts the patient via his external device 60 whether the patient would like to continue adjusting the pole configuration position, and/or whether the patient would additionally like to experiment with changing other stimulation parameters (A, PW, F) that do not affect the position at which the pole configuration is applied in the electrode array. If the patient wishes no further adjustments, the algorithm 160 can end. Else, the algorithm 160 returns to step 210, which moves the pole configuration again, and allows the process to repeat.

Returning to step 216, if the patient's pain score is low, the algorithm in step 218 can compare the ECAP measurements Sim to the ECAP baselines Sib retrieved earlier (step 170, Fig. 11) to see whether they are equal in step 220. As one skilled will appreciate, determining that these measured and baseline values are equal does not necessarily mean that they are exactly equal. Instead, "equal" in this context can include some margin of error, which can depend on the ECAP features that are compared. For example, the peaks in the measured and baseline ECAPs can be said to be equal if they differ in time (e.g., Δt; Fig. 10A) by a very small amount (e.g., 0.01 ms); the ECAP durations (e.g., Fig 10C) can be said to be equal if they are if they again differ by a small amount (e.g., 0.1 ms), etc.

If the measured and baseline values are equal, the algorithm 160 will assume, despite the patient's low pain score, that the position of the pole configuration appears to be optimized, and may notify the patient of that fact in step 222. However, the patient may still benefit from adjustments to stimulation, even if such adjustments move the position of the pole configuration. Therefore, the patient may be prompted at step 222 to see whether they would like to adjust any stimulation parameters, or if the algorithm 160 should end (not shown). Such adjustments at step 222 can be freely chosen by the patient, perhaps as assisted by the use of other optimization algorithms beyond the scope of this disclosure. In any event, if the patient's symptoms improved as reflected in an entered pain score (step 224), the algorithm 160 may once again take ECAP measurements (step 225), and prompt the patient whether to store such measurements as the new baselines (step 227), similar to what occurred in step 226 above. If the patient's symptoms aren't improved at step 224 although the pole position appears optimized, the patient may continue to iteratively adjust stimulation parameters that don't affect pole configuration position at step 229, hopefully eventually leading to improved symptoms, and to steps 225 and 227 as discussed above.

Returning to step 220, if ECAP measurements do not equal the ECAP baselines, the algorithm 160 will proceed to adjust the pole configuration to yet another new position. This can occur in a number of ways, and can be assisted by the use of counters (Count 1 and Count 2), which keep the algorithm 160 from running in an infinite loop in case a match between the ECAP measurements Sim and the ECAP baselines Sib cannot be established. Assume for example that Count 1 has a threshold of four, and Count 2 has a threshold of three. If in step 220 a match cannot be established, Count 1 is incremented (from zero to one) in step 230, and is compared to its threshold (of four) in step 232. Because Count 1's threshold is not met at this point, the algorithm 160 returns to step 210, thus allowing the algorithm to move the pole configuration to another new position, allowing the process to repeat. This is useful, because it allows the algorithm 160 to iteratively try a number of new pole configuration positions, have the patient rank them (step 216), and have new ECAP measurements Sim compared to the baselines Sib (step 220).

Once Count 1's threshold is met-i.e., after four new pole configuration positions are assessed but with no match at step 220-the algorithm 160 proceeds to step 234 where Count 2 is incremented (to one) and Count 1 is reset (to zero). Count 2 is compared to its threshold (three) in step 236, and if this threshold is not met, the patient's external device can prompt whether the patient wishes to continue adjusting the position of the pole configuration in step 238. Alternatively, the algorithm 160 in step 238 may also allow the patient to adjust other stimulation parameters that do not affect the position of the pole configuration (e.g., A, PW, F). If the patient desires to continue moving the pole configuration, the algorithm 160 may continue to try up to four more pole configuration positions, as set by Count 1's threshold. Otherwise, the algorithm 160 may end (not shown).

Eventually, Count 2's threshold may be met, which would mean in this example that the algorithm 160 has tried twelve different pole configuration positions, as set by Count 1 and Count 2's thresholds (four times three), but still no match has been determined at step 220. At this point, the algorithm 160 may conclude that it is unable to fully optimize the position of the pole configuration by matching the ECAP measurements Sim to the ECAP baselines Sib, as shown at step 240. As also shown, the algorithm 160-having at this point assessed twelve different pole configurations-may prompt the patient if he wishes to use the pole configuration that was best, even if Sim for that pole configuration does not equal Sib. A "best" pole configuration can be determined in different ways. For example, a best pole configuration can comprise that for which the patient entered the best (highest) pain score (step 216). Alternatively, a best pole configuration may be one in which Sim is closest to Sib, even if not "equally." Sim can be "closer to" Sib if one or more of S1m, S2m, or S3m's features (e.g., time of arrival, duration, amplitude, etc.) is closer to the corresponding baseline S1b, S2b, S3b. In this sense, even if the algorithm 160 was not able to adjust the pole configuration position to the point where Sim equals Sib, the algorithm 160 can still improve the therapy provided to the patient, and therefore compensate for electrode array 17 movement. This being said, the hope would be that in some iteration-that for some pole configuration position-the ECAP measurements Sim and Sib would be made equal at step 220.

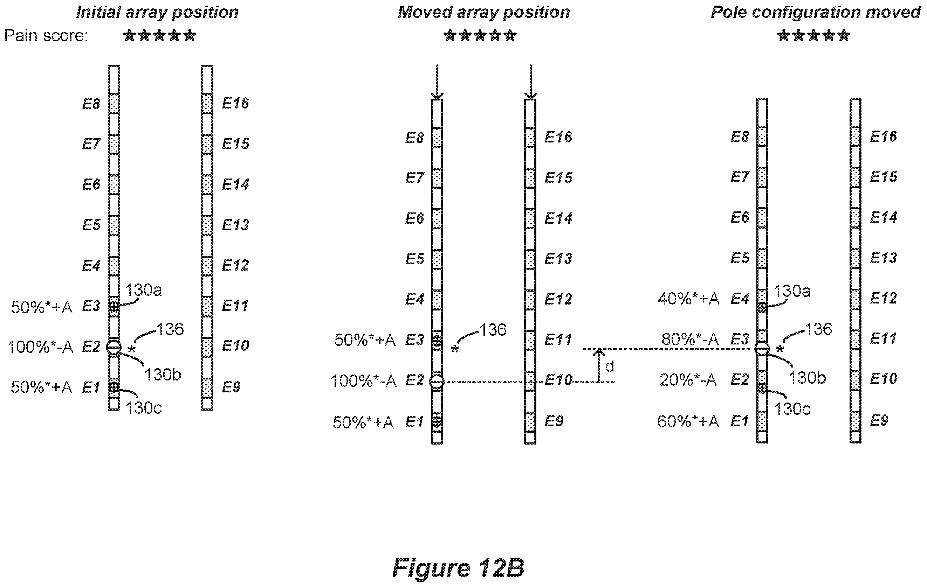

Figure 12B shows the effect of use of the algorithm 160 to compensate for electrode array movement. The left shows the pole configuration (tripole) introduced earlier, which is at this point optimized to recruit stimulation target 136 in the patient's tissue. At this point, the patient reports a high pain score (five stars). In the middle, it is seen that the electrode array has moved downwards. As a result, the pole configuration is no longer well aligned with the stimulation target 136, and thus the patient's pain score has dropped (three stars). This low pain score, as noted earlier, can prompt use of the algorithm 160, which may result in movement of the position of the pole configuration, as shown to the right. In this case, the pole configuration has been moved up in the electrode array by a distance d to compensate for the downward movement of the electrode array, such that the pole configuration again aligns with the stimulation target 136. Notice also how electrode configuration algorithm 150 (Fig. 8) has operated to select different electrodes with particular polarities and current percentages to place the cathode pole 130b and anodes poles 130a and 130c at their new moved positions.

How the pole configuration can be moved at step 210 (Fig. 12A) can occur in different manners. In one example, the patient may be prompted on his external device 60 how to move the pole configuration, i.e., in what direction (up, down, left, or right) and by what distance. In this regard, the patient's external device may contain a GUI similar to that shown in Figure 8. The pole configuration can also be iteratively moved automatically by the algorithm 160. In one example, in a first iteration of step 210, the pole configuration could be moved 1 mm to the right in the electrode array 17; in a next iteration it could be moved 1 mm to the left; then 1 mm up, and 1 mm down. Once the best of these random positions is determined (i.e., where Sim best equals Sib), the process can be repeated until eventually a best pole configuration position is determined. Having said this, the pole configuration need not necessarily be moved at random in step 210, and instead can be automatically moved in a more informed manner using data gleaned from the comparison of the measured and baseline ECAPs.

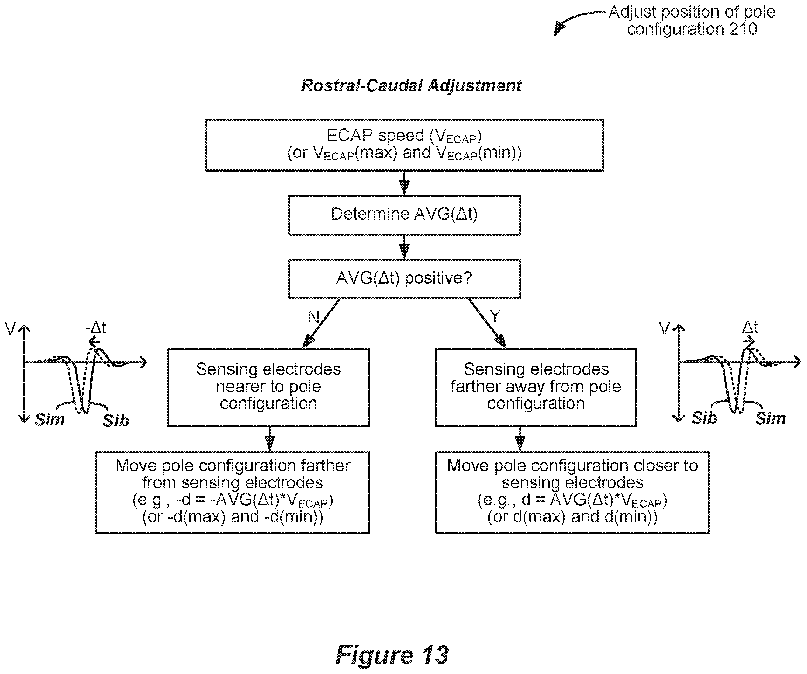

Figure 13 shows a first example of how the pole configuration can be moved in an informed manner in step 210. This example is particularly useful in adjusting the pole configuration rostrally or caudally in the electrode array 17. In this example, the speed of neural conduction of ECAPs in the patient's tissue, V_{ECAP}, can be used to determine a distance d that the pole configuration should be moved in the electrode array, and in what direction (rostrally or caudally). V_{ECAP} may be known a priori, or may have been measured earlier by the ECAP algorithm 124, for example, by knowing the spacing between the sensing electrodes S1, S2, and S3 in the array, and sensing the time difference at which the ECAP passes each.

The measured ECAPs Sim and the baseline ECAPs Sib as determined earlier in the algorithm 160 (Fig. 11) can be assessed to determine whether the measured ECAPs Sim are arriving at the sensing electrodes earlier in time than the baseline ECAPs Sib would reflect (-Δt), or whether the measured ECAPs Sim are arriving at the sensing electrodes later in time than the baseline ECAPs would reflect (Δt). In this regard, note that a Δt will exist for each of the sensing electrodes used (e.g., between S1m and S1b, between S2m and S2b, etc.), and it can be logical to take the average of these time shifts (AVG(Δt)). If AVG(Δt) is positive, this means that the sensing electrodes have moved farther away from the pole configuration than they used to be. If AVG(Δt) is negative, the sensing electrodes have moved nearer to the pole configuration. This could result for example if the electrode array 17 has moved by becoming straighter or more bent in the patient.

As Figure 13 shows, if the sensing electrodes have moved farther away from the pole configuration, the pole configuration can be moved closer to the sensing electrodes, and V_{ECAP} and AVG(Δt) can inform the algorithm 160 as to the distance d that the pole configuration should be moved-i.e., d = AVG(Δt) * V_{ECAP}. Conversely, if the sensing electrodes have moved nearer to the pole configuration, the pole configuration can be moved farther from the sensing electrodes by a distance -d = -AVG(Δt) * V_{ECAP}. In short, at step 210, a direction and distance in which the pole configuration can be moved can be established by a comparison of Sim to Sib. Alternatively, if the speed of the ECAP may fall within a range, from V_{ECAP}(min) to V_{ECAP}(max), then a range of distances, from d(max) to d(min), can be established, thus allowing different distances in this range to be tried at step 210 in different iterations of the algorithm 160.

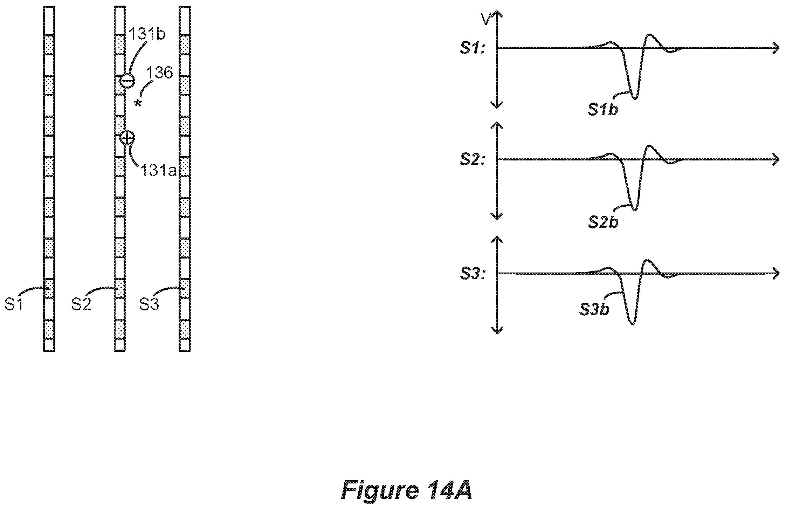

Figures 14A and 14B show another example of how the pole configuration can be moved by algorithm 160 in an informed manner in step 210, and in particular as to how the pole configuration can be moved in a medio-lateral manner, that is, from left to right in the electrode array. In this example, and as shown in Figure 14A, sensing electrodes S 1, S2, and S3 are provided medio-laterally in the electrode array. The electrode array 17 in this example is shown as comprising three percutaneous leads, but a paddle lead 19 (Fig. 1) could also have been used to illustrate this example. As before, a pole configuration has been established to recruit a stimulation target 136, and in this example, the pole configuration comprises a bipole with an anode pole 131a and a cathode pole 131b. Again, these poles 131 can be positioned anywhere in the electrode array through use of the electrode configuration algorithm 150 (Fig. 8). An ECAP as sensed at sensing electrodes S1-S3 spaced medio-laterally in the electrode array is shown in Figure 14A as well, which may comprise ECAP baselines Sib used by the algorithm 160. Because the sensing electrodes are generally equidistant from the pole configuration, the ECAP arrives at each sensing electrode at essentially the same time. Additionally, the amplitude of the ECAP is generally the same at each sensing electrode, although in reality sensing electrodes in better rostral-caudal alignment with the pole configuration (e.g., sensing electrode S2) may have larger amplitudes.

Figure 14B shows the effect in measured ECAPs if the electrode array moves medio-laterally. For example, suppose as shown that the middle lead in the electrode array has moved to the left in the patient's tissue as shown. This will bring the pole configuration closer to S1, and further from S3. Therefore, measured ECAP S1m will increase in amplitude from its baseline S1b, while measured ECAP S3m will decrease from its baseline S3b as shown. This can indicate electrode array movement; although the examples illustrates movement of only part of the array (the middle lead), the technique can still be applicable if the entire array moves as well.

As was the case for rostral-caudal adjustment of pole configuration position in Figure 13, the measured ECAPs and baseline ECAPs can be assessed to determine if the electrode array (or a part of the electrode array) has moved, which can inform how to move the pole configuration in step 210. A simple example is illustrated in which the algorithm 160 inquires as to which measured ECAPs are larger or smaller in amplitude compared to their baselines. For example, if S1m < S1b and S3m > S3b, then the pole configuration has moved closer to S3 and further from S1. In other words, the pole configuration has moved to the right. Therefore, at step 210, the algorithm 160 can move the pole configuration to the left to compensate and to try and bring Sim back equal to or closer to Sib. By contrast, and as shown in Figure 14B, if S1m > S1b and S3m < S3b, then the pole configuration has moved closer to S1 and further from S3. In other words, the pole configuration has moved to the left. Therefore, at step 210, the algorithm 160 can move the pole configuration to the right to compensate, and as shown in Figure 14B, the pole configuration has been moved a distance d to the right.

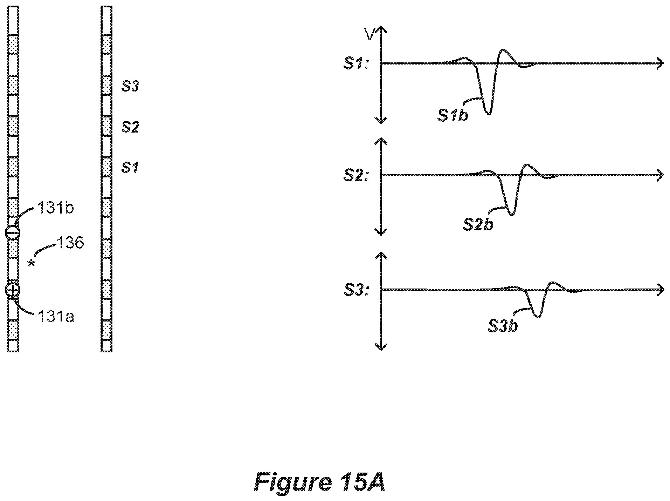

Figure 15A shows a different example in which the sensing electrodes are on a different lead in the electrode array 17 from the electrodes involved in producing the pole configuration. As shown, electrodes on the left lead in the array are used to form the pole configuration, again a bipole, which bipole is recruiting a stimulation target 136, The right lead includes sensing electrodes, and the waveform to the right show ECAP baselines resulting form the stimulation. In this example, sensing electrode S1 is closest to the pole configuration, and hence the ECAP arrives at this electrode first, and with a relatively high amplitude (S1b). Sensing electrodes S3 is furthest from the pole configuration, and hence the ECAP arrives at this electrode last, and with a relatively small amplitude (S3b).

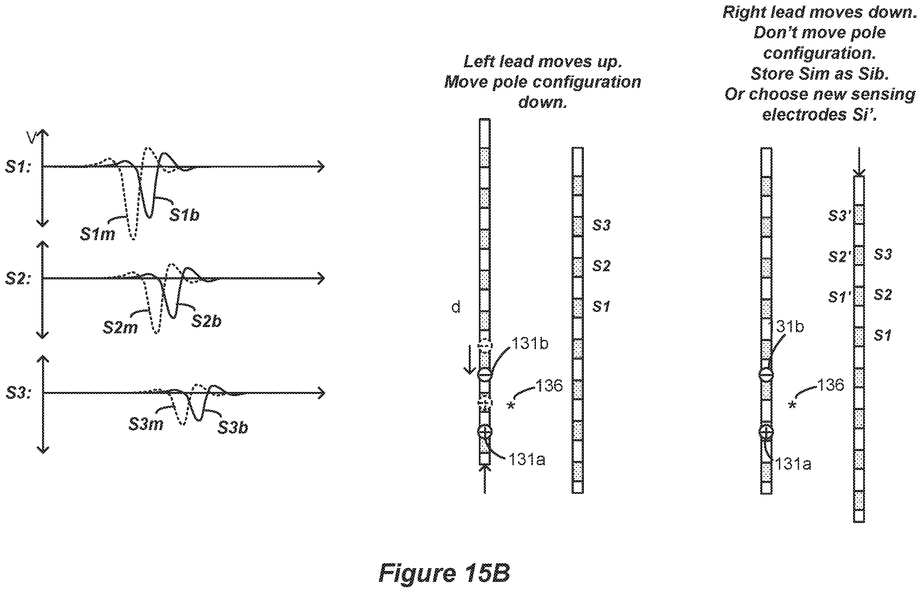

Figure 15B shows possible results when the leads in the electrode array 17 move relative to each other. The waveforms show ECAP measurements Sim that are both earlier in time and larger in amplitude when compared to their ECAP baseline counterparts Sib. From this, the algorithm 160 may conclude that sensing electrodes have moved closer to the stimulating electrodes used to form the pole configuration (although it may not yet be clear as to which of the leads might have moved). This can be useful in step 210 when deciding how to move the pole configuration. For example, the algorithm 160 may infer that the left lead used to form the pole configuration has moved upward in the tissue while the right lead with the sensing electrodes has stayed stationary. If so, at step 210, the algorithm 160 can move the pole configuration downward on the left lead as shown so that the pole configuration better aligns with the stimulation target 136. Presumably the algorithm would also see in this circumstance that such movement of the pole configuration would tend to bring the measured ECAPs Sim back to (or closer to) their baseline levels Sib.

However, it is also possible given the ECAP measurements Sim that the right lead with the sensing electrodes has moved downward in the tissue while the left lead used to form the pole configuration has stayed stationary. In this circumstance, there is no need to adjust the position of the pole configuration, as it is still properly aligned to recruit the stimulation target 136. The algorithm 160 may not initially be able to discern between these two possible movements of the leads, but can still try moving the pole configuration downward. However, moving the pole configuration downward would move the pole configuration further away from the stimulation target, which should cause the patient's pain score to get worse (lower). In this circumstance, and although not shown, the algorithm 160 could be modified to not move the pole configuration, but instead may take other actions. For example, the algorithm 160 could store the new ECAP measurements Sim as the ECAP baselines Sib to update them as would be warranted given the shift in position of the right lead. The algorithm 160 could also choose new sensing electrodes Si' which would be farther away from the stimulating electrodes to readjust the relative distance between them, and again use measured ECAPs Sim at these new sensing electrodes as new baselines Sib.

Although described in the context of ECAPs, it should be noted that the disclosed technique can be used with any type of neural response to stimulation. Further, while the disclosed technique is borne out of concern for adjusting a patient's therapy in light of electrode array movement, the disclosed technique can be used to adjust a patient's therapy in any context.

## Claims

1. A system, comprising:
an implantable stimulation device (100) comprising an electrode array configured to provide stimulation to a patient's tissue;
an external device (60, 70) configured to communicate with the implantable stimulation device; and
an algorithm configured to operate at least in part in the implantable stimulation device, wherein the algorithm is configured to
(a) provide therapeutic stimulation to the patient's tissue using a pole configuration at a position in the electrode array, wherein the therapeutic stimulation has been determined for the patient to be effective to recruit a stimulation target in the spinal column;
(b) measure a neural response from the pole configuration at the position at one or more sensing electrodes in the electrode array as a measured response;
(c) compare the measured response at each of the at least one sensing electrodes to a baseline response at a corresponding one of the sensing electrodes; and
(d) if the measured response does not equal the baseline response, adjust the position of the pole configuration in the electrode array and repeating steps (a)-(c) until the measured response equals or is closer to the baseline response.

2. The system of claim 1, wherein the algorithm is configured to operate wholly in the implantable stimulation device.

3. The system of claim 1, wherein steps (a) and (b) are configured to operate in the implantable stimulation device, and wherein steps (c) and (d) are configured to operate in the external device.

4. The system of any of claims 1-3, wherein in step (d) the position of the pole configuration is adjusted until the measured response equals the baseline response.

5. The system of any of claims 1-4, wherein in step (c) at least one feature of the measured response at each of the sensing electrodes is compared to the at least one feature of the baseline response at the corresponding one of the sensing electrodes.

6. The system of claim 5, wherein the at least one feature comprises one or more of a time or speed at which the measured response and the baseline response arrives at each of the sensing electrodes, a duration of the measured response and the baseline response at each of the sensing electrodes, or an amplitude of the measured response and the baseline response at each of the sensing electrodes.

7. The system of any of claims 1-6, wherein the baseline response is stored in a database, wherein the baseline response comprises a neural response measured at the one or more sensing electrodes in the electrode array in response to providing the therapeutic stimulation using the pole configuration at an initial position in the electrode array.

8. The system of claim 7, wherein the algorithm starts in step (a) by providing the therapeutic stimulation to the patient's tissue using the pole configuration at the initial position in the electrode array.

9. The system of claims 7 or 8, wherein the database is stored in the implantable stimulation device.

10. The system of claims 7 or 8, wherein the database is stored in the external device.

11. The system of any of claims 7-10, wherein the database comprises a neural response measured at the one or more sensing electrodes at a plurality of different postures of the patient.

12. The system of claim 11, wherein the implantable stimulation device includes a means for determining a posture of the patient, wherein in step (c) the measured response at each of the at least one sensing electrodes is compared to a baseline response at a corresponding one of the sensing electrodes as stored for the determined posture.

13. The system of any of claims 1-12, wherein the external device is configured to provide a Graphical User Interface (GUI), and wherein the GUI is configured to receive an indication of a symptom of a patient, wherein the algorithm is automatically initiated if the indication is not suitable relative to a threshold.

14. The system of any of claims 1-13, wherein in step (d) the measured response and the baseline response are used to determine a direction for adjusting the position of the pole configuration, and/or a distance for adjusting the position of the pole configuration in the direction.

15. A system, comprising:
an implantable stimulation device (100) comprising an electrode array configured to provide stimulation to a patient's tissue;
an external device (60, 70) configured to communicate with the implantable stimulation device; and
an algorithm configured to operate at least in part in the implantable stimulation device, wherein the algorithm is configured to
(a) provide therapeutic stimulation to the patient's tissue using a pole configuration at a position in the electrode array, wherein the therapeutic stimulation has been determined for the patient to be effective to recruit a stimulation target in the spinal column;
(b) measure a neural response from the pole configuration at the position at one or more sensing electrodes in the electrode array as a measured response;
(c) assess the consistency of the measured response at each of the at least one sensing electrodes to a baseline response at a corresponding one of the sensing electrodes;
(d) use the assessed consistency as determined in step (c) to determine whether to:
(i) adjust a position of the pole configuration in the electrode array, or
(ii) adjust other stimulation parameters of the pole configuration that do not affect the position of the pole configuration; and
(e) provide the adjustment of (i) or (ii) depending on the determination of step (d).

## Patentansprüche

1. System, das aufweist:
eine implantierbare Stimulationsvorrichtung (100), die eine Elektrodenanordnung aufweist, die konfiguriert ist, eine Stimulation für das Gewebe eines Patienten bereitzustellen;
eine externe Vorrichtung (60, 70), die konfiguriert ist, mit der implantierbaren Stimulationsvorrichtung zu kommunizieren; und
einen Algorithmus, der konfiguriert ist, zumindest teilweise in der implantierbaren Stimulationsvorrichtung zu arbeiten, wobei der Algorithmus konfiguriert ist:
(a) eine therapeutische Stimulation für das Gewebe des Patienten unter Verwendung einer Polkonfiguration an einer Position in der Elektrodenanordnung bereitzustellen, wobei festgestellt worden ist, dass die therapeutische Stimulation für den Patienten wirksam ist, ein Stimulationsziel in der Wirbelsäule anzusprechen;
(b) eine neurale Reaktion von der Polkonfiguration an der Position an einer oder mehreren Abtastelektroden in der Elektrodenanordnung als eine gemessene Reaktion zu messen;
(c) die gemessene Reaktion an jeder der mindestens einen Abtastelektrode mit einer Basislinienreaktion an einer entsprechenden Abtastelektrode zu vergleichen; und
(d) wenn die gemessene Reaktion nicht der Basislinienreaktion entspricht, die Position der Polkonfiguration in der Elektrodenanordnung einzustellen und die Schritte (a)-(c) zu wiederholen, bis die gemessene Reaktion der Basislinienreaktion entspricht oder ihr näher kommt.

2. System nach Anspruch 1, wobei der Algorithmus konfiguriert ist, vollständig in der implantierbaren Stimulationsvorrichtung zu arbeiten.

3. System nach Anspruch 1, wobei die Schritte (a) und (b) konfiguriert sind, in der implantierbaren Stimulationsvorrichtung zu arbeiten, und wobei die Schritte (c) und (d) konfiguriert sind, in der externen Vorrichtung zu arbeiten.

4. System nach einem der Ansprüche 1 bis 3, wobei in Schritt (d) die Position der Polkonfiguration eingestellt wird, bis die gemessene Reaktion gleich der Basislinienreaktion ist.

5. System nach einem der Ansprüche 1 bis 4, wobei in Schritt (c) mindestens ein Merkmal der gemessenen Reaktion an jeder der Abtastelektroden mit dem mindestens einen Merkmal der Basislinienreaktion an der entsprechenden Abtastelektrode verglichen wird.

6. System nach Anspruch 5, wobei das mindestens eine Merkmal eines oder mehreres aufweist von: einer Zeit oder Geschwindigkeit, zu der bzw. mit der die gemessene Reaktion und die Basislinienreaktion an jeder der Abtastelektroden eintreffen, einer Dauer der gemessenen Reaktion und der Basislinienreaktion an jeder der Abtastelektroden oder einer Amplitude der gemessenen Reaktion und der Basislinienreaktion an jeder der Abtastelektroden.

7. System nach einem der Ansprüche 1 bis 6, wobei die Basislinienreaktion in einer Datenbank gespeichert wird, wobei die Basislinienreaktion eine neurale Reaktion aufweist, die an der einen oder den mehreren Abtastelektroden in der Elektrodenanordnung als Reaktion auf das Bereitstellen der therapeutischen Stimulation unter Verwendung der Polkonfiguration an einer Anfangsposition in der Elektrodenanordnung gemessen wird.

8. System nach Anspruch 7, wobei der Algorithmus in Schritt (a) beginnt, indem dem Gewebe des Patienten die therapeutische Stimulation unter Verwendung der Polkonfiguration an der Anfangsposition in der Elektrodenanordnung bereitgestellt wird.

9. System nach Anspruch 7 oder 8, wobei die Datenbank in der implantierbaren Stimulationsvorrichtung gespeichert ist.

10. System nach Anspruch 7 oder 8, wobei die Datenbank in der externen Vorrichtung gespeichert ist.

11. System nach einem der Ansprüche 7 bis 10, wobei die Datenbank eine neurale Reaktion aufweist, die an der einen oder den mehreren Abtastelektroden bei mehreren unterschiedlichen Körperhaltungen des Patienten gemessen wurde.

12. System nach Anspruch 11, wobei die implantierbare Stimulationsvorrichtung eine Einrichtung zum Bestimmen einer Körperhaltung des Patienten enthält, wobei im Schritt (c) die gemessene Reaktion an jeder der mindestens einen Abtastelektrode mit einer Basislinienreaktion an einer entsprechenden Abtastelektrode verglichen wird, die für die bestimmte Körperhaltung gespeichert ist.

13. System nach einem der Ansprüche 1 bis 12, wobei die externe Vorrichtung konfiguriert ist, eine grafische Benutzeroberfläche (GUI) bereitzustellen, und wobei die GUI konfiguriert ist, eine Anzeige für ein Symptom eines Patienten zu empfangen, wobei der Algorithmus automatisch eingeleitet wird, wenn die Anzeige in Bezug auf einen Schwellenwert nicht geeignet ist.

14. System nach einem der Ansprüche 1 bis 13, wobei in Schritt (d) die gemessene Reaktion und die Basislinienreaktion verwendet werden, um eine Richtung zum Einstellen der Position der Polkonfiguration und/oder einen Abstand zum Einstellen der Position der Polkonfiguration in der Richtung zu bestimmen.

15. System, das aufweist:
eine implantierbare Stimulationsvorrichtung (100), die eine Elektrodenanordnung aufweist, die konfiguriert ist, eine Stimulation für das Gewebe eines Patienten bereitzustellen;
eine externe Vorrichtung (60, 70), die konfiguriert ist, mit der implantierbaren Stimulationsvorrichtung zu kommunizieren; und
einen Algorithmus, der konfiguriert ist, zumindest teilweise in der implantierbaren Stimulationsvorrichtung zu arbeiten, wobei der Algorithmus konfiguriert ist:
(a) eine therapeutische Stimulation für das Gewebe des Patienten unter Verwendung einer Polkonfiguration an einer Position in der Elektrodenanordnung bereitzustellen, wobei festgestellt worden ist, dass die therapeutische Stimulation für den Patienten wirksam ist, ein Stimulationsziel in der Wirbelsäule anzusprechen;
(b) eine neuralen Reaktion von der Polkonfiguration an der Position an einer oder mehreren Abtastelektroden in der Elektrodenanordnung als gemessene Reaktion zu messen;
(c) die Übereinstimmung der gemessenen Reaktion an jeder der mindestens einen Abtastelektrode mit einer Basislinienreaktion an einer entsprechenden der Abtastelektroden zu bewerten;
(d) die bewertete Übereinstimmung, die in Schritt (c) bestimmt wurde, dazu zu verwenden, um festzustellen, ob:
(i) eine Position der Polkonfiguration in der Elektrodenanordnung angepasst werden soll, oder
(ii) andere Stimulationsparameter der Polkonfiguration angepasst werden sollen, die die Position der Polkonfiguration nicht beeinflussen; und
(e) die Einstellung von (i) oder (ii) abhängig von der Bestimmung im Schritt (d) bereitzustellen.

## Revendications

1. Système, comprenant :
un dispositif de stimulation implantable (100) comprenant un réseau d'électrodes configuré pour appliquer une stimulation aux tissus d'un patient ;
un dispositif externe (60, 70) configuré pour communiquer avec le dispositif de stimulation implantable ; et
un algorithme configuré pour fonctionner au moins en partie dans le dispositif de stimulation implantable, dans lequel l'algorithme est configuré pour
(a) appliquer une stimulation thérapeutique aux tissus du patient en utilisant une configuration de pôle à une position dans le réseau d'électrodes, dans lequel la stimulation thérapeutique a été déterminée pour le patient comme étant efficace pour recruter une cible de stimulation dans la colonne vertébrale ;
(b) mesurer une réponse neuronale de la configuration de pôle à la position au niveau d'une ou plusieurs électrodes de détection dans le réseau d'électrodes en tant que réponse mesurée ;
(c) comparer la réponse mesurée au niveau de chacune des une ou plusieurs électrodes de détection à une réponse de référence au niveau d'une électrode de détection correspondante des électrodes de détection ; et
(d) si la réponse mesurée n'est pas égale à la réponse de référence, régler la position de la configuration de pôle dans le réseau d'électrodes et répéter les étapes (a) à (c) jusqu'à ce que la réponse mesurée soit égale à la réponse de référence ou plus proche de celle-ci.

2. Système selon la revendication 1, dans lequel l'algorithme est configuré pour fonctionner entièrement dans le dispositif de stimulation implantable.

3. Système selon la revendication 1, dans lequel les étapes (a) et (b) sont configurées pour fonctionner dans le dispositif de stimulation implantable, et dans lequel les étapes (c) et (d) sont configurées pour fonctionner dans le dispositif externe.

4. Système selon l'une des revendications 1 à 3, dans lequel à l'étape (d), la position de la configuration de pôle est réglée jusqu'à ce que la réponse mesurée soit égale à la réponse de référence.

5. Système selon l'une des revendications 1 à 4, dans lequel à l'étape (c), au moins une caractéristique de la réponse mesurée au niveau de chacune des électrodes de détection est comparée à l'au moins une caractéristique de la réponse de référence au niveau de l'électrode de détection correspondante des électrodes de détection.

6. Système selon la revendication 5, dans lequel l'au moins une caractéristique comprend un ou plusieurs parmi un temps ou une vitesse auxquels la réponse mesurée et la réponse de référence arrivent à chacune des électrodes de détection, une durée de la réponse mesurée et de la réponse de référence au niveau de chacune des électrodes de détection, ou une amplitude de la réponse mesurée et de la réponse de référence au niveau de chacune des électrodes de détection.

7. Système selon l'une des revendications 1 à 6, dans lequel la réponse de référence est stockée dans une base de données, dans lequel la réponse de référence comprend une réponse neuronale mesurée au niveau des une ou plusieurs électrodes de détection du réseau d'électrodes en réponse à l'application de la stimulation thérapeutique en utilisant la configuration de pôle à une position initiale dans le réseau d'électrodes.

8. Système selon la revendication 7, dans lequel l'algorithme commence à l'étape (a) en appliquant la stimulation thérapeutique aux tissus du patient en utilisant la configuration de pôle à la position initiale dans le réseau d'électrodes.

9. Système selon les revendications 7 ou 8, dans lequel la base de données est stockée dans le dispositif de stimulation implantable.

10. Système selon les revendications 7 ou 8, dans lequel la base de données est stockée dans le dispositif externe.

11. Système selon l'une des revendications 7 à 10, dans lequel la base de données comprend une réponse neurale mesurée au niveau des une ou plusieurs électrodes de détection dans une pluralité de postures différentes du patient.

12. Système selon la revendication 11, dans lequel le dispositif de stimulation implantable comporte un moyen pour déterminer une posture du patient, dans lequel à l'étape (c), la réponse mesurée au niveau de chacune des une ou plusieurs électrodes de détection est comparée à une réponse de référence au niveau d'une électrode de détection correspondante des électrodes de détection telle que stockée pour la posture déterminée.

13. Système selon l'une des revendications 1 à 12, dans lequel le dispositif externe est configuré pour fournir une interface utilisateur graphique (GUI), et dans lequel la GUI est configurée pour recevoir une indication d'un symptôme d'un patient, dans lequel l'algorithme est automatiquement initié si l'indication n'est pas appropriée par rapport à un seuil.

14. Système selon l'une des revendications 1 à 13, dans lequel à l'étape (d), la réponse mesurée et la réponse de référence sont utilisées pour déterminer une direction pour régler la position de la configuration de pôle, et/ou une distance pour régler la position de la configuration de pôle dans la direction.

15. Système, comprenant :
un dispositif de stimulation implantable (100) comprenant un réseau d'électrodes configuré pour appliquer une stimulation aux tissus d'un patient ;
un dispositif externe (60, 70) configuré pour communiquer avec le dispositif de stimulation implantable ; et
un algorithme configuré pour fonctionner au moins en partie dans le dispositif de stimulation implantable, dans lequel l'algorithme est configuré pour
(a) appliquer une stimulation thérapeutique aux tissus du patient en utilisant une configuration de pôle à une position dans le réseau d'électrodes, dans lequel la stimulation thérapeutique a été déterminée pour le patient comme étant efficace pour recruter une cible de stimulation dans la colonne vertébrale ;
(b) mesurer une réponse neuronale de la configuration de pôle à la position au niveau d'une ou plusieurs électrodes de détection dans le réseau d'électrodes en tant que réponse mesurée ;
(c) évaluer la cohérence de la réponse mesurée au niveau de chacune des une ou plusieurs électrodes de détection par rapport à une réponse de référence au niveau d'une électrode de détection correspondante des électrodes de détection ;
(d) utiliser la cohérence évaluée telle qu'elle a été déterminée à l'étape (c) pour déterminer s'il faut :
(i) régler une position de la configuration de pôle dans le réseau d'électrodes, ou
(ii) régler d'autres paramètres de stimulation de la configuration de pôle qui n'affectent pas la position de la configuration de pôle ; et
(e) proposer le réglage (i) ou (ii) en fonction de la détermination à l'étape (d).
